(19)

Europäisches
Patentamt
European
Patent Office
Office européen
des brevets

(11) **EP 3 181 698 A1**

(12) **EUROPEAN PATENT APPLICATION**

(43) Date of publication:
21.06.2017 Bulletin 2017/25

(51) Int Cl.:
*C12Q 1/68* (2006.01)        *C12N 15/11* (2006.01)
*C12N 5/0735* (2010.01)

(21) Application number: 15200521.1

(22) Date of filing: 16.12.2015

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **European Molecular Biology Laboratory (EMBL)
69117 Heidelberg (DE)**

(72) Inventors:
• **Sladitschek, Hanna
69121 Heidelberg (DE)**
• **Neveu, Pierre
69120 Heidelberg (DE)**

(74) Representative: **Krauss, Jan
Boehmert & Boehmert
Anwaltspartnerschaft mbB
Patentanwälte Rechtsanwälte
Pettenkoferstrasse 20-22
80336 München (DE)**

(54) **MICRORNA MIR-142 AS STEM CELL MARKER**

(57)   The present invention relates to a method for detecting a pluripotent stem cell (PSC) in an undifferentiated state, comprising analysing miRNA 142 expression in a PSC, wherein an increase of the expression level of miRNA 142 is indicative for an undifferentiated state, when compared to a differentiating or differentiated PSC. Further provided is a method for maintaining a pluripotent stem cell (PSC) in an undifferentiated state, comprising promoting the expression or overexpression of miRNA 142 in said stem cell and a method for inducing or promoting the differentiation of a pluripotent stem cell (PSC), comprising inhibiting the expression or function of miRNA 142 in said stem cell. The invention also provides a method for identifying a compound that modulates the differentiation of a pluripotent stem cell (PSC), comprising a) contacting an PSC with a potential modulator compound, and b) detecting the expression and/or function of miRNA 142 in said PSC in response to said modulator compound, wherein a change in the expression and/or function of miRNA 142 is indicative for a compound that modulates the differentiation in said PSC.

## Description

The present invention relates to a method for detecting a pluripotent stem cell (PSC) in an undifferentiated state, comprising analysing miRNA 142 expression in a PSC, wherein an increase of the expression level of miRNA 142 is indicative for an undifferentiated state, when compared to a differentiating or differentiated PSC. Further provided is a method for maintaining a pluripotent stem cell (PSC) in an undifferentiated state, comprising promoting the expression or overexpression of miRNA 142 in said stem cell and a method for inducing or promoting the differentiation of a pluripotent stem cell (PSC), comprising inhibiting the expression or function of miRNA 142 in said stem cell. The invention also provides a method for identifying a compound that modulates the differentiation of a pluripotent stem cell (PSC), comprising a) contacting an PSC with a potential modulator compound, and b) detecting the expression and/or function of miRNA 142 in said PSC in response to said modulator compound, wherein a change in the expression and/or function of miRNA 142 is indicative for a compound that modulates the differentiation in said PSC.

## Background of the invention

Stem cells respond to internal and external cues by self-renewal or commitment to a differentiated fate (North et al., 2007; Jiang et al., 2009; Medema and Vermeulen, 2011; Kueh et al., 2013; Blanpain and Fuchs, 2014). Current models suggest that this balance is controlled in vivo by stem cell niches (Scadden, 2006; Voog and Jones, 2010; Simons and Clevers, 2011) and in vitro by an appropriate growth factor environment (Murry and Keller, 2008; Pera and Tam, 2010).

Mouse embryonic stem cells (mESCs) constitute a powerful system to study the molecular mechanism of fate decisions in controlled in vitro environment (Rue and Martinez Arias, 2015). mESCs are continuous cell lines derived from the inner cell mass of the blastocyst (Evans and Kaufman, 1981; Martin, 1981). These cells can be propagated indefinitely in vitro while maintaining their pluripotency, i.e. the capacity to give rise to derivatives of all three germ layers and germ cells both in vitro and in vivo.

microRNAs (miRNAs) are small non-coding RNAs that act as post-transcriptional regulators of gene expression (Bartel, 2009). A growing body of evidence suggests that miRNAs act as key players in stem cell homeostasis (Neumüller et al., 2008; Foronda et al., 2014) and cell fate decisions (Chen et al., 2004; Li and Carthew, 2005; Johnston et al., 2005; Wang et al., 2007; Yi et al., 2008; Schwamborn et al., 2009). However, which miRNAs control stem cell pluripotency decisions and by what mechanism they carry out this important function is largely unknown.

Isobe et al. (in: miR-142 regulates the tumorigenicity of human breast cancer stem cells through the canonical WNT signaling pathway; eLife 2014;3:e01977, November 18, 2014) disclose that miR-142 and miR-150 are upregulated in human breast cancer stem cells (BCSCs) as compared to the non-tumorigenic breast cancer cells. In this study, we report that miR-142 efficiently recruits the *APC* mRNA to an RNA-induced silencing complex, activates the canonical WNT signaling pathway in an APC-suppression dependent manner, and activates the expression of miR-150. Enforced expression of miR-142 or miR-150 in normal mouse mammary stem cells resulted in the regeneration of hyperproliferative mammary glands in vivo. Knockdown of endogenous miR-142 effectively suppressed organoid formation by BCSCs and slowed tumor growth initiated by human BCSCs in vivo.

Abdul Razak et al. (in: Abdul Razak SR, Baba Y, Nakauchi H, Otsu M, Watanabe S. DNA Methylation Is Involved in the Expression of miR-142-3p in Fibroblasts and Induced Pluripotent Stem Cells. Stem Cells Int. 2014; 2014:101349. Epub 2014 Dec 2) have been analyzing comprehensive expression patterns of microRNA in human and mouse embryonic stem and induced pluripotent stem cells. They determined microRNAs specifically expressed in these pluripotent stem cells, and miR-142-3p is one of such microRNAs. miR-142-3p is expressed at higher levels in induced pluripotent stem cells relative to fibroblasts in mice. Level of expression of miR142-3p decreased during embryoid body formation from induced pluripotent stem cells. Loss-of-function analyses of miR-142-3p suggested that miR-142-3p plays roles in the proliferation and differentiation of induced pluripotent stem cells. CpG motifs were found in the 5' genomic region of the miR-142-3p; they were highly methylated in fibroblasts, but not in undifferentiated induced pluripotent stem cells. Treating fibroblasts with 5-aza-2'-deoxycytidine increased the expression of miR-142-3p significantly and reduced methylation at the CpG sites, suggesting that the expression of miR-142-3p is suppressed by DNA methylation in fibroblasts. Luciferase analysis using various lengths of the 5' genomic region of miR142-3p indicated that CpGs in the proximal enhancer region may play roles in suppressing the expression of miR-142-3p in fibroblasts.

Lu et al. (in: Xinyan Lu et al. miR-142-3p regulates the formation and differentiation of hematopoietic stem cells in vertebrates Cell Research (2013) 23:1356-1368; published online 29 October 2013) show that in zebrafish and mouse, *miR-142-3p* is specifically expressed in hematopoietic stem cells (HSCs). Knockdown of *miR-142a-3p* in zebrafish led to a reduced population of HSCs in the aorta-gonad-mesonephros (AGM) region as well as T-cell defects in the thymus.

Shende et al. (in: Shende VR, Neuendorff N, Earnest DJ (2013) Role of miR-142-3p in the Post-Transcriptional Regulation of the Clock Gene Bmal1 in the Mouse SCN. PLoS ONE 8(6): e65300) suggest that miR-142-3p may play a role in the post-transcriptional modulation of *Bmal1* and its oscillatory regulation in molecular feedback loops mediating SCN circadian function.

US 2014-0288149 relates to compositions and methods for treating an autoimmune disease in a subject, comprising administering to the subject a compound comprising an oligonucleotide, wherein the oligonucleotide comprises a first strand that consists of 8 to 200 nucleosides, and wherein the first strand comprises a region that is identical to at least 8 contiguous nucleotides of miR-142, preferably miR-142 is miR-142-5p or miR-142-3p. WO 2014/140856 provides methods of treating various conditions using miR-142, miR-142 mimics, and antagonists of miR-142.

US 2014-0336239 relates to methods and compositions for modulating the differentiation of a myeloid derived suppressor cell (MDSC). In particular, described herein are miR-142 polynucleotides and miR-223 polynucleotides that can be used to modulate differentiation of MDSCs. Increased differentiation of a MDSC population, or cells within an MDSC population, can be achieved by increasing the miR-142 and/or miR-223 polynucleotides in a MDSC.

WO 2010/056737 discloses methods and compositions for treating a patient having, suspected of having, or at risk of developing cancer by targeting cancer stem cells. hsa-miR-142-3p, hsa-miR-142-5p are mentioned.

It is an object of the present invention to provide new tools and methods for using miR-142, in particular with respect to the control of the differentiation of stem cells. Other aspects and objects will become apparent for the person of skill upon reading the following more detailed description of the invention.

According to a first aspect thereof, the above objects have been solved by the present invention by providing a method for detecting a pluripotent stem cell (PSC) in an undifferentiated state, comprising analysing miRNA 142 expression in an PSC, wherein an increase of the expression level of miRNA 142 is indicative for an undifferentiated state, when compared to a differentiating or differentiated PSC.

In the context of the present invention, the tem "pluripotent stem cell (PSC)" shall include both embryonic stem cells, ESCs, and induced pluripotent stem cells (iPSC), as the properties of both types of cells are very similar, if not identical (see, e.g. Zhengping Jiang, Yanmei Han and Xuetao Cao, Induced pluripotent stem cell (iPSCs) and their application in immunotherapy. Cellular & Molecular Immunology (2014) 11, 17-24; Vimal K. Singh, Manisha Kalsan, Neeraj Kumar, Abhishek Saini, and Ramesh Chandra, Induced pluripotent stem cells: applications in regenerative medicine, disease modeling, and drug discovery. Front Cell Dev Biol. 2015; 3: 2).

According to a second aspect thereof, the above objects have been solved by the present invention by providing a method for maintaining a pluripotent stem cell (PSC) in an undifferentiated state, comprising promoting the expression or overexpression of miRNA 142 in said stem cell.

According to a third aspect thereof, the above objects have been solved by the present invention by providing a method for inducing or promoting the differentiation of a pluripotent stem cell (PSC), comprising inhibiting the expression or function of miRNA 142 in said stem cell.

According to a fourth aspect thereof, the above objects have been solved by the present invention by providing an in vitro method for identifying a compound that modulates the differentiation of a pluripotent stem cell (PSC), comprising a) contacting a PSC with a potential modulator compound, and b) detecting the expression and/or function of miRNA 142 in said

PSC in response to said modulator compound, wherein a change in the expression and/or function of miRNA 142 is indicative for a compound that modulates the differentiation in said PSC.

According to a fifth aspect thereof, the above objects have been solved by the present invention by providing a pharmaceutical preparation comprising a compound that promotes the expression or overexpression of miRNA 142 in a cell for use in the treatment of cancer..

The inventors found that PSCs with high miR-142 expression are irresponsive to differentiation signals while cells with low miR-142 expression can respond to differentiation cues.

The inventors used a single-cell miRNA activity reporter to identify miR-142 that is bimodally expressed in mESCs under pluripotency-maintaining conditions. miR-142 expression levels stratify mESCs with indistinguishable expression of pluripotency markers into two distinct subpopulations: mESCs with low miR-142 levels are amenable to signal-induced differentiation, while cells with high miR-142 levels are irresponsive to differentiation cues. Using quantitative experiments and simulations, they showed that mESCs switch stochastically between the high and low miR-142 states. Dissecting the molecular mechanism, they found that miR-142 represses the activation of KRAS/ERK signaling in a double-negative feedback loop that creates a bistable system. Thus, the self-generated miR-142 two-state system functions to maintain a stem cell reservoir that is protected from differentiation signals from the environment.

The bimodally expressed microRNA miR-142 defines two stochastically interconverting states of pluripotent embryonic stem cells: a state competent to differentiate (low miR-I42 expression) and a state irresponsive to differentiation signals akin to a stem cell reservoir (high miR-142 expression). miR-142 targets Kras and represses the activation of MAPK/ERK and AKT/PKB. This reveals a novel mechanism to maintain a stem cell reservoir buffered against fluctuating signaling environments.

The results explain why clonal stem cells fail to uniformly differentiate even in a uniform differentiation culture environment. Therefore, these findings are of fundamental interest to regenerative medicine applications where induced pluripotent stem cells failing to differentiate pose a risk of tumor development.

According to the invention, miR-142 can be used as marker to identify stem cells in tissues, and isolate them for use in regenerative medicine applications. Suppression of miR-142 can be used to obtain more uniform differentiation from induced pluripotent cell lines or adult stem cell lines. miR-142 targets an oncogene (Kras) and suppresses two key signaling pathways involved in cancer development and progression, MAPK/ERK and AKT/PKB. miR-142 can therefore be seen as a tumor suppressor. In addition, miR-142 expression or lack of thereof can be used as a diagnostic marker to classify the tumor dependency on KRAS, ERK and AKT to decide further treatment options.

The inventors have generated a transgenic mouse model ubiquitously expression a miR-142 sensor. This will enable to characterize the expression of miR-142 in the developing embryo and adult tissues. miR-142 expressing cells will be characterized for their stem cell properties by RNA sequencing and functional assays. Proliferation and survival will be assessed upon miR-142 delivery. miR-142 expression will be assessed in human induced pluripotent cell lines.

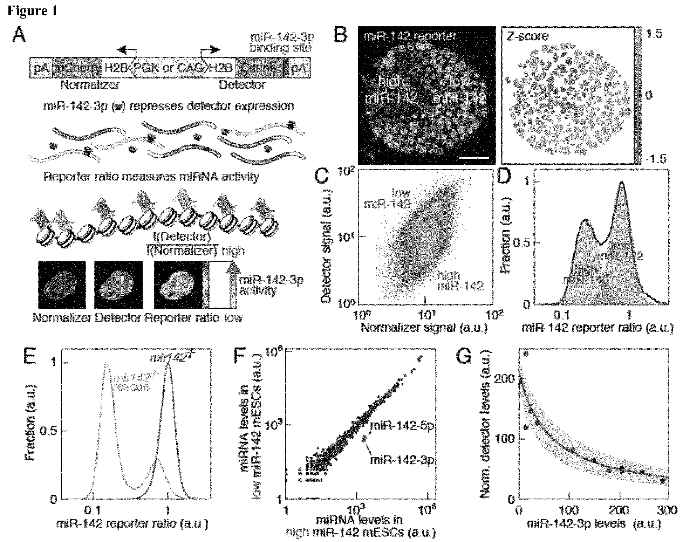

The inventors identified an increase of the expression level of miRNA 142 in undifferentiated or undifferentiating ESCs. The analysis showed a 10-fold increase in the expression levels of miR-142-3p and miR-142-5p, the two mature forms of the miR-142 stem loop, in "high" miR-142 mESCs compared to "low" miR-142 mESCs (Figure 1F). Thus, preferred is a method according to the present invention, wherein said increase is between about 2 to 20-fold or more, preferably between about 5 to 15-fold, more preferred at about 8 to 12-fold, and most preferred at about 10-fold, when compared to a "low" miR-142 ESC, preferably when compared to a differentiating or differentiated ESC.

In the context of the present invention, "about" shall mean +/- 10% of a given value, unless indicated otherwise. The methods of the present invention can be performed in vivo or in vitro, in particular in laboratory animals, or in culture.

As used herein, the term "miR-142" encompasses miR-142-5p, miR-142-3p, and pre-miR-142. Thus, preferred is the method according to the present invention, wherein said miRNA 142 is selected from miR-142-3p and miR-142-5p.

As used herein, "miR-142-5p" refers to a microRNA having the sequence 5'-CAUAAAGUAGAAAGCACUACU-3' (SEQ ID NO: 1). In some embodiments, the matching region of miR-142-5p comprises nucleotides 1 to 9, nucleotides 1 to 8, nucleotides 1 to 7, nucleotides 2 to 9, nucleotides 2 to 8, or nucleotides 2 to 7.

As used herein, "miR-142-3p" refers to a microRNA having the sequence 5'-UGUAGUGUUUCCUACUUUAUGGA-3' (SEQ ID NO: 2). In some embodiments, the matching region of miR-142-3p comprises nucleotides 1 to 9, nucleotides 1 to 8, nucleotides 1 to 7, nucleotides 2 to 9, nucleotides 2 to 8, or nucleotides 2 to 7.

As used herein, "pre-miR-142" refers to a stem-loop having the sequence 5'-GACAGUGCAGUCACCCAUAAAGUAGAAAGCACUACUAACAGCACUGGAGGGUG UAGUGUUUCCUACUUUAUGGAUGAGUGUACUGUG-3' (SEQ ID NO: 3).

The methods according to the present invention can be used to detect, identify and/or manipulate stem cells. Preferred are embryonic stem cells, in particular stem cells that have been prepared or obtained by a method which does not involve the destruction of a mammalian, such as human, embryo. More preferred are pluripotent stem cells, and/or stem cells that are derived from reprogrammed somatic cells. Thus, further preferred is a method according to the present invention, wherein said undifferentiated ESC is a pluripotent stem cell, optionally staining positive for the pluripotency marker OCT4.

Preferred is the method according to the present invention, wherein said PSC is a mammalian PSC, such as a rat, mouse, goat, rabbit, sheep, horse, monkey or human PSC, more preferred is a mouse, rat or human PSC.

Preferred is the method according to the present invention, wherein said PSC is present in a biological sample. The sample can be any kind of biological sample, e.g. body fluid, comprising stem cell(s). The sample can also be cancer or tumor tissue comprising stem cells.

Another aspect of the present invention the relates to a method for maintaining a pluripotent stem cell (PSC) in an undifferentiated state according to the present invention, comprising expressing or overexpressing miRNA 142 in said stem cell. Preferred is the method according to the present invention, wherein said miRNA 142 is selected from miR-142-3p and miR-142-5p.

Preferred is the method according to the present invention for maintaining a pluripotent stem cell (PSC) in an undifferentiated state, comprising promoting the expression or overexpression of miRNA 142 in said stem cell. Preferred is a method according to the present invention, wherein said promoting (increase) of the expression or overexpression is between about 2 to 20-fold or more, preferably between about 5 to 15-fold, more preferred at about 8 to 12-fold, and most preferred at about 10-fold, when compared to a "low" miR-142 PSC, preferably when compared to a differentiating or differentiated PSC.

Said promoting preferably comprises introducing an inducer of the expression of miRNA 142, an expression vector and/or an oligonucleotide comprising a region that is homologous or preferably identical to the nucleotides of miR-142 into said PSC. Preferred is the method according to the present invention, wherein said miRNA 142 is selected from miR-142-3p and miR-142-5p. As used herein, the term "homologous" refers a nucleic acid which is at least 85%, at least 90%, at least 95%, or 100% (identical) to the nucleotides of miR-142.

In some embodiments, oligonucleotides are provided, wherein the oligonucleotides comprise a region that is homologous to at least 8, at least 9, at least 10, at least 11, at least 12, at least 13, at least 14, at least 15, at least 16, at least 17, at least 18, at least 19, or at least 20 nucleotides of miR-142, of miR-142-5p, of miR-142-3p or of pre-miR-142.

In some embodiments, oligonucleotides are provided, wherein the oligonucleotides comprise a region that is identical to at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, at least 50, at least 55, at least 60, at least 70, or at least 80 nucleotides of pre-miR-142.

In some embodiments, an oligonucleotide that comprises a region that is identical to miR-142 is a miR-142 mimic. In some such embodiments, the oligonucleotide may further comprise a complementary strand. In some such embodiments, the oligonucleotide may be referred to as a double-stranded miR-142 mimic. Double-stranded microRNA mimics are commercially available, e.g., from Qiagen (miScript), Sigma Aldrich, Invitrogen (mirVana), and Thermo Scientific (miRIDIAN microRNA mimics). In some embodiments, the strand comprising the region that is identical to miR-142 and the complementary strand are part of a single oligonucleotide. In some embodiments of double-stranded miR-142 mimics, the double-stranded region of the mimic is 15 to 30 nucleotides in length.

In some embodiments, a miR-142 mimic is an siRNA. A siRNA, or small interfering RNA, is an RNA comprising a double-stranded region of 15 to 25 base pairs in length. In some embodiments, an siRNA comprises a 5'-phosphate and a 3'-hydroxyl. In some embodiments, an siRNA comprises a two-base overhang (i.e., single-stranded region) on the 3' end of one or both of the RNA strands. One strand of the siRNA is an oligonucleotide (such as an RNA oligonucleotide) that comprise a region that is identical to at least 8, at least 9, at least 10, at least 11, at least 12, at least 13, at least 14, at least 15, at least 16, at least 17, at least 18, at least 19, or at least 20 nucleotides of miR-142. The oligonucleotide, in some embodiments, comprises a region that is identical to the seed match region of miR-142. In some embodiments, such an siRNA targets the same site(s) in the same genes as miR-142, and is therefore considered to be a mimic.

In some embodiments, a miR-142 mimic is a shRNA. A shRNA, or short hairpin RNA, comprises a single RNA strand that is self-complementary over at least a portion of the RNA. In some embodiments, an shRNA is delivered to a subject by administering a vector comprising a coding sequence for the shRNA, such as in gene therapy. See, e.g., Xiang et al., 2006, Nature Biotech., 24: 697-702; Senzer et al., 2012, Mol. Therap., 20: 679-686; US 2010-0299771; US 2012-0004283.

In some embodiments, a vector encoding a shRNA may be administered in order to increase miR-142 levels in a particular cell, cell type, tissue, or subject. In some embodiments, a vector comprising the coding sequence for the miR-142 pre-miRNA or pri-miRNA may be administered in order to increase miR-142 levels in a particular cell, cell type, tissue, or subject. Recombinant expression vectors for miRNAs are known in the art, and recombinant lentiviral expression vectors are the most widely used delivery vehicle for miRNA delivery due to their high efficiency transduction and stable integration. Other routes of delivery (e.g. as particles) are also known to the person of sikill (Powell SK, Rivera-Soto R, Gray SJ. Viral expression cassette elements to enhance transgene target specificity and expression in gene therapy. Discov Med. 2015 Jan;19(102):49-57; Xie J, Burt DR, Gao G. Adeno-associated virus-mediated microRNA delivery and therapeutics. Semin Liver Dis. 2015 Feb;35(1):81-8; Wang H, Jiang Y, Peng H, Chen Y, Zhu P, Huang Y. Recent progress in microRNA delivery for cancer therapy by non-viral synthetic vectors; Adv Drug Deliv Rev. 2015 Jan;81:142-60; Attar R, Sajjad F, Qureshi MZ, Tahir F, Hussain E, Fayyaz S, Farooqi AA. TRAIL based therapy: overview of mesenchymal stem cell based delivery and miRNA controlled expression of TRAIL. Asian Pac J Cancer Prev. 2014;15(16):6495-7; Tay FC, Lim JK, Zhu H, Hin LC, Wang S. Using artificial microRNA sponges to achieve microRNA loss-of-function in cancer cells. Adv Drug Deliv Rev. 2015 Jan;81:117-27; Lemoinne S, Thabut D, Housset C, Moreau R, Valla D, Boulanger CM, Rautou PE. The emerging roles of microvesicles in liver diseases. Nat Rev Gastroenterol Hepatol. 2014 Jun;11(6):350-61).

As used herein, the term "oligonucleotide" refers to an oligomer comprising modified and/or unmodified nucleosides. Modified nucleosides may comprise modified sugar moieties and/or modified nucleobase moieties. Further, an oligonucleotide may comprise modified internucleoside linkages, unmodified internucleoside linkages, or both modified and unmodified internucleoside linkages.

Another aspect of the present invention then relates to a method for inducing or promoting the differentiation of a pluripotent stem cell (PSC), comprising inhibiting the expression or function of miRNA 142 in said stem cell. Preferred is a method according to the present invention, wherein said inhibiting (decrease) of the expression or overexpression is between about 2 to 20-fold or more, preferably between about 5 to 15-fold, more preferred at about 8 to 12-fold, and most preferred at about 10-fold, when compared to a "high" miR-142 PSC, preferably when compared to a non-differentiating or undifferentiated PSC.

Said inhibiting or reducing preferably comprises introducing an inhibitor of the expression or function of miRNA 142 into said PSC, such as, for example an oligonucleotide comprising a region that is complementary to the nucleotides of miR-142. Preferred is the method according to the present invention, wherein said miRNA 142 is selected from miR-142-3p and miR-142-5p.

As used herein, the term "complementary" refers to the ability of a nucleotide on a first nucleic acid to pair with a nucleotide on a second nucleic acid. When a region of a nucleic acid is "complementary" to a region, or set of contiguous nucleotides, of a second nucleic acid, the region may be at least 85%, at least 90%, at least 95%, or 100% complementary to the region, or set of contiguous nucleotides, of the second nucleic acid. Thus, for example, unless otherwise indicated, a region of a first nucleic acid that is complementary to 10 contiguous nucleotides of a second nucleic acid may comprise one mismatch relative to the 10 contiguous nucleotides of the second nucleic acid. An oligonucleotide comprising that region is considered to be complementary to 10 contiguous nucleotides of the second nucleic acid. When there are no mismatches, in some embodiments, the first nucleic acid is said to be "100% complementary" or "fully complementary" to the region, or set of contiguous nucleotides, of the second nucleic acid.

In some embodiments, oligonucleotides are provided, wherein the oligonucleotides comprise a region that is complementary to at least 8, at least 9, at least 10, at least 11, at least 12, at least 13, at least 14, at least 15, at least 16, at least 17, at least 18, at least 19, or at least 20 nucleotides of miR-142 of miR-142-5p, of miR-142-3p or of pre-miR-142.

In some embodiments, an oligonucleotide that comprises a region that is complementary to miR-142 is a single-stranded oligonucleotide. In some embodiments, an oligonucleotide that comprises a region that is complementary to miR-142 is referred to as a miR-142 antagonist or a miR-142 antisense. Single-stranded microRNA inhibitors are commercially available, for example, from Qiagen (miScript miRNA inhibitors). In some embodiments, the oligonucleotide that comprises a region that is complementary to miR-142 is a hairpin microRNA inhibitor, which comprises a self-complementary region such that the oligonucleotide folds into a hairpin. Commercial microRNA hairpin inhibitors are available, e.g., from Thermo Scientific (miRIDIAN microRNA hairpin inhibitors).

In some embodiments, an oligonucleotide comprises at least one modified nucleoside and/or modified internucleoside linkage. In some embodiments, such modifications may increase the binding affinity and specificity of an oligonucleotide for its target nucleic acid as compared to oligonucleotides that contain only deoxyribonucleotides, and may allow for the use of shorter polynucleotides or for shorter regions of complementarity between the oligonucleotide and the target nucleic acid. In some embodiments, such modifications may (or may also) increase the nuclease resistance of the oligonucleotide, improving the pharmacokinetics such that lower doses of the oligonucleotide may be needed to therapeutic effect.

In some embodiments, an oligonucleotide includes one or more modified nucleosides, wherein each modified nucleoside comprises a modified nucleobase moiety and/or a modified sugar moiety. In some embodiments, an oligonucleotide comprises one or more modified internucleoside linkages, one or more unmodified internucleoside linkages, or a combination of modified and unmodified internucleoside linkages.

Nonlimiting exemplary modified nucleosides having modified nucleobase moieties include nucleosides comprising 5-methylcytosine, isocytosine, pseudoisocytosine, 5-bromouracil, 5-propynyluracil, 6-aminopurine, 2-aminopurine, inosine, diaminopurine, 2-chloro-6-aminopurine, xanthine and hypoxanthine. Nonlimiting exemplary modified nucleosides having modified sugar moieties include nucleosides comprising 2'-substituted sugars, such as 2'-O-alkyl-ribose sugars, 2'-amino-deoxyribose sugars, 2'-fluoro-deoxyribose sugars, 2'-fluoroarabinose sugars, and 2'-O-methoxyethyl-ribose (2'MOE) sugars, and bicyclic sugars, such as locked nucleic acid ("LNA"). In some embodiments, modified sugars are arabinose sugars, or d-arabino-hexitol sugars.

In some embodiments, an oligonucleotide comprises one or more backbone modifications such as peptide nucleic acids (PNA; e.g., an oligomer including nucleobases linked together by an amino acid backbone). Other backbone modifications include, but are not limited to, phosphorothioate linkages, phosphodiester modified nucleic acids, combinations of phosphodiester and phosphorothioate nucleic acid, methylphosphonate, alkylphosphonates, phosphate esters, alkylphosphonothioates, phosphoramidates, carbamates, carbonates, phosphate triesters, acetamidates, carboxymethyl esters, methylphosphorothioate, phosphorodithioate, p-ethoxy, and combinations thereof.

One skilled in the art can design a suitable oligonucleotide for an intended application using the knowledge in the art. Nonlimiting exemplary descriptions of oligonucleotides that antagonize microRNAs or mimic microRNAs (such as siRNA) and considerations for designing such oligonucleotides, including suitable modified nucleosides and internucleoside linkages, are described, for example, in US 2011-0166198; US 8,017,763; US 8,173,611; WO 2005-013901; US 2012-0184596; US 2009-270481; EP 1984382; EP1824975; US 7,834,170; WO 2012-149646; Breving et al. Int J Biochem Cell Biol. 2010 August; 42(8):1316-29; van Rooij et al., Circ Res. 2012 Feb. 3; 110(3):496-507; Iorio et al., EMBO Mol. Med. 2012 March; 4(3): 143-159; Frieden, M. et al. (2008) Curr. Pharm. Des. 14(11):1138-1142, each of which is incorporated by reference herein in its entirety for any purpose.

According to the invention, miR-142 can also be used as a marker to identify stem cells that are irresponsive to differentiation in regenerative medicine applications and therefore achieve more efficient differentiation. miR-142 inhibition can in addition be used to aid differentiation. In addition, miR-142 expression can be used to isolate stem cells from patient samples. miR-142 or mimics thereof could be used as anti-cancer agent. Finally miR-142 expression or the lack thereof can be used as a diagnostic marker in cancer to classify the cellular responsiveness to and dependency on AKT and ERK signaling.

According to another aspect thereof, the present invention provides an method for identifying a compound that modulates the differentiation of a pluripotent stem cell (PSC), comprising a) contacting an PSC with a potential modulator compound, and b) detecting the expression and/or function of miRNA 142 in said PSC in response to said modulator compound, wherein a change in the expression and/or function of miRNA 142 is indicative for a compound that modulates the differentiation in said PSC. Optionally, said compound is identified as being suitable for modulating the differentiation of an embryonic stem cell (PSC).

More preferred is a method for detecting and/or identifying according to the present invention, wherein said compound is selected from the group consisting of a peptide library, a combinatory library, a cell extract, in particular a plant cell extract, a "small molecular drug", an antisense oligonucleotide, an siRNA, and an antibody or fragment thereof (such as Fab or scFv, and the like).

Preferred is a method for detecting and/or identifying according to the present invention, further comprising testing said compound(s) as detected/identified for its activity on the differentiation of PSCs, and/or the expression and/or function of miRNA 142. Respective assays are known to the person of skill, and can be taken from the respective literature. Said modulator either induces or reduces the expression and/or function of miRNA 142 in said PSC. Preferred is the induction.

The screening method can be performed in vivo (e.g. in test animals as disclosed herein) or in in vitro models, such as stem cell culture systems, as it is known to the person of skill, and can be autoimated as well (Tabata et al. Multiparametric Phenotypic Screening System for Profiling Bioactive Compounds Using Human Fetal Hippocampal Neural Stem/Progenitor Cells. J Biomol Screen. 2015 Oct;20(9):1074-83).

Preferred is a method for detecting and/or identifying according to the present invention, wherein steps a) and b) are repeated, and, optionally, chemically modifying said compound before said repeating. The thus identified candidate compound can then, in a preferred embodiment, modified in a further step. Modification can be effected by a variety of methods known in the art, which include, without limitation, the introduction of novel side chains or the exchange of functional groups like, for example, introduction of halogens, in particular F, Cl or Br, the introduction of lower alkyl groups, preferably having one to five carbon atoms like, for example, methyl, ethyl, n-propyl, isopropyl, n-butyl, isobutyl, tert-butyl, n-pentyl or isopentyl groups, lower alkenyl groups, preferably having two to five carbon atoms, lower alkynyl groups, preferably having two to five carbon atoms or through the introduction of, for example, a group selected from the group consisting of $NH_2$, $NO_2$, OH, SH, NH, CN, aryl, heteroaryl, COH or COOH group. The thus modified binding substances are than individually tested with a method of the present invention. If needed, the steps of selecting the candidate compound, modifying the compound, and testing compound can be repeated a third or any given number of times as required. The above described method is also termed "directed evolution" since it involves a multitude of steps including modification and selection, whereby binding compounds are selected in an "evolutionary" process optimizing its capabilities with respect to a particular property, e.g. its ability to act on miRNA 142 expression and/or activity.

Another aspect of the present invention relates to a method for manufacturing a pharmaceutical composition for use in the differentiation of PSCs, comprising the steps of: performing a method for detecting and/or identifying according to the present invention, and formulating said compound as detected and identified into a pharmaceutical composition.

In a further embodiment of the method of the present invention, the compound identified as outlined above, which may or may not have gone through additional rounds of modification and selection, is admixed with suitable auxiliary substances and/or additives. Such substances comprise pharmacological acceptable substances, which increase the stability, solubility, biocompatibility, or biological half-life of the interacting compound like, for example, pharmaceutically acceptable salts, esters or salts of such esters, or bioequivalent compounds thereof, admixed, encapsulated, conjugated or otherwise associated with liposomes, polymers, receptor targeted molecules, oral, rectal, topical or other formulations that assist uptake, distribution and/or absorption, and optionally further comprising penetration enhancers, carrier compounds, and/or transfection agents. Carriers, excipients and strategies to formulate a pharmaceutical composition, are well known to the person of skill and described in the respective literature.

Administration of an agent, e.g., a compound can be accomplished by any method which allows the agent to reach the target cells. These methods include, e.g., injection, implantation, transfection, or any other method of administration where access to the target cells by the agent is obtained. The compound can be suspended in liquid, e.g., in dissolved or colloidal form. The liquid can be a solvent, partial solvent or non-solvent. In many cases, water or an organic liquid can be used.

Yet another aspect of the present invention is directed at a pharmaceutical composition, obtainable or obtained by a method according to the method as herein.

As mentioned above, miR-142 targets an oncogene (Kras) and suppresses two key signaling pathways involved in cancer development and progression, MAPK/ERK and AKT/PKB. miR-142 can therefore be seen as a tumor suppressor. Another aspect of the invention thus relates to the pharmaceutical composition as described herein comprising a compound that promotes the expression or overexpression of miRNA 142 in a cell for use in medicine, in particular for use in the treatment of cancer and/or cancer stem cells. Another aspect of the invention thus relates to a method for treating cancer, comprising administering to a cancer cell a pharmaceutically effective amount of said pharmaceutical composition. Preferably, said pharmaceutically effective amount suppresses at least one of the signaling pathways involved in cancer development and progression, MAPK/ERK and AKT/PKB in said cancer, in particular in the difficult to treat cancer stem cells.

It should be understood that an amount of a therapeutic compound, such as a nucleic acid that is provided to a cancer cell or organism is an "effective amount," which refers to an amount needed (or a sufficient amount) to achieve a desired goal, such as inducing a particular cellular characteristic(s) or reducing cancer growth or killing cancer cells and/or alleviating symptoms associated with a cancer.

Preferred is the use according to the present invention, wherein said promoting (increase) of the expression or overexpression is between about 2 to 20-fold or more, preferably between about 5 to 15-fold, more preferred at about 8 to 12-fold, and most preferred at about 10-fold, when compared to a non-treated cancer cell. Said promoting preferably comprises introducing an inducer of the expression of miRNA 142 as described above, an expression vector as described above and/or an oligonucleotide as described above, comprising a region that is homologous or preferably identical to the nucleotides of miR-142 into said cancer cell. Preferred is the method according to the present invention, wherein said miRNA 142 is selected from miR-142-3p and miR-142-5p. Most preferred is a pharmaceutical composition as described herein comprising miRNA 142, miR-142-3p and/or miR-142-5p for use in the treatment of cancer.

Other compounds that promote the expression or overexpression of miRNA 142 in a cell for use in medicine can be selected from the group consisting of a peptide library, a combinatory library, a cell extract, in particular a plant cell extract, a "small molecular drug", an antisense oligonucleotide, an siRNA, and an antibody or fragment thereof (such as Fab or scFv, and the like). Prefered are therapeutic nucleic acids (e.g. oligonucleotides) or synthetic or nonsynthetic molecules that correspond (in particularly functionally) to a miRNA sequence as disclosed herein.

Routes of administration will vary, naturally, with the location and nature of the cancer or stem cell to be targeted, and include, e.g., intradermal, subcutaneous, regional, parenteral, intravenous, intramuscular, intranasal, systemic, and oral administration and formulation. Injection or perfusion of a therapeutic nucleic acid is specifically contemplated for discrete, solid, accessible precancers or cancers, or other accessible target areas. Local, regional, or systemic administration also may be appropriate.

The inventors's results support a model in which a double-negative feedback loop between Kras/ERK-signaling and miR-142 produces a bistable system trapping mESCs in either a "low" miR-142 state that has a high level of ERK/AKT activity and is competent to differentiate or on the other hand a "high" miR-142 state that has a low level of ERK/AKT activity and is blocked from differentiation. High miR-142 levels hereby act as a red traffic light that prevents cells from transducing differentiation signals into gene expression programs driving exit from pluripotency and initiation of differentiation. Low miR-142 levels correspond to the green light that allows the initiation of differentiation.

The mESC heterogeneity as described here is caused by the action of a single microRNA. Such cell-to cell variation is inaccessible to single-cell mRNA-Seq approaches and undetectable by traditional biochemical assays which yield population-average data. The findings add a previously uncharacterized layer of cellular heterogeneity that is upstream of the so far reported mESC heterogeneity at the level of transcription factors (Chambers et al., 2007; Singh et al., 2007; Toyooka et al., 2008).

The differential signaling pathway activation in the "high" and "low" miR-142 state explains the functional phenotypic differences as observed. High AKT activation in "low" miR-142 mESCs is likely to render them more clonogenic, as AKT signaling is known to facilitate cell survival (Paling et al., 2004). The higher sensitivity of "low" miR-142 mESCs to instructive differentiation cues can be readily explained by their nonrepressed activation of ERK signaling, a major transmitter of differentiation stimuli (Kunath et al., 2007).

Indeed, the genetic deletion of mir142 led to increased levels of ERK and AKT activation, which explains the superior performance of mir142-/- mESCs in differentiation and clonogenicity assays. The action of miR-142 in controlling competence for differentiation might be of functional relevance in in vivo development or in the maintenance of adult stem cell compartments. Indeed, miR-142 is required to specify hemangioblast fate (Nimmo et al., 2013) and enforced miR-142 expression impairs macrophage differentiation in vivo (Sonda et al., 2013). Moreover, mir142-/- mice have numerous hematopoietic defects: impaired lymphopoiesis (Kramer et al., 2015), megakaryopoiesis (Chapnik et al., 2014) and CD34+ dendritic cell homeostasis (Mildner et al., 2013).

Stochasticity in cell fate decisions (Losick and Desplan, 2008) has been described to play an important role ranging from bacterial differentiation (Süel et al., 2006) via cancer development (Gupta et al., 2011) to mammalian stem cells (Klein et al., 2010). However, the mir142 stochastic switch as uncovered here is not a fate switch but rather a mechanism that determines the competence of stem cells to respond to external stimuli. Cells that have entered the "high" miR-142 state are deaf to instructive differentiation signals and are therefore locked in an undifferentiated state until they interconvert into the "low" miR-142 state. Maintaining a stem cell subpopulation in a "high" miR-142 state irresponsive to differentiation cues is a mechanism to protect a reservoir of stem cells from differentiation, which can slowly interconvert into a responsive "low" miR-142 state without being completely diminished due to the high rate of self renewal. Such a buffering mechanism safeguards developmental plasticity in the face of fluctuating or noisy environmental conditions and would prevent depletion of a stem cell reservoir even upon long periods of exposure to differentiation signals.

The results explain why clonal stem cells fail to uniformly differentiate even in a uniform differentiation culture environment (Canham et al., 2010). Therefore, these findings are of fundamental interest to regenerative medicine applications where induced pluripotent stem cells failing to differentiate pose a risk of tumor development (Cohen and Melton, 2011).

The present invention will now be illustrated further in the following non-limiting examples, with reference to the accompanying figures. For the purposes of the invention, all publications, patent applications, patents, and other references mentioned herein are incorporated by reference in their entireties.

Figure 1 shows that the bimodal expression of miR-142 distinguishes two states in mESCs. (A) Scheme of the experimental approach to monitor miRNA activity in single cells. (B) Confocal section of a single cell-derived mESC colony stably expressing the miR-142-3p activity reporter and corresponding Z-score of the reporter ratio. As individual mESCs are not motile within a colony, sister lineages are spatially clustered. Bar: 50 mm. (C) Detector and normalizer expression in a clonal mESC population stably expressing the miR-142-3p reporter. (D) Distribution of the miR-142-3p reporter ratio in a clonal mESC culture. Two log-normal distributions ("high" miR-142 activity state: gray shaded area; "low" miR-142 activity state: gray shaded area) approximated well the experimental data (black line). (E) Distribution of the miR-142-3p reporter ratio in mir142-/- mESCs (line) and mir142-/- mESCs transgenic for the mir142-hosting lincRNA driven by its own promoter (mir142-/- rescue, gray line). (F) Deep sequencing analysis of miRNA expression levels in FACS-purified "high" and "low" miR-142 states. Levels of the two mature forms of miR-142, miR-142-3p and miR-142-5p are highlighted in gray. (G) Hill's equation with noncooperative binding approximated well detector mRNA expression levels as a function of miR-142-3p levels (dark dots: experimental data measured by deep sequencing; gray line: fit; shaded area: fit confidence interval).

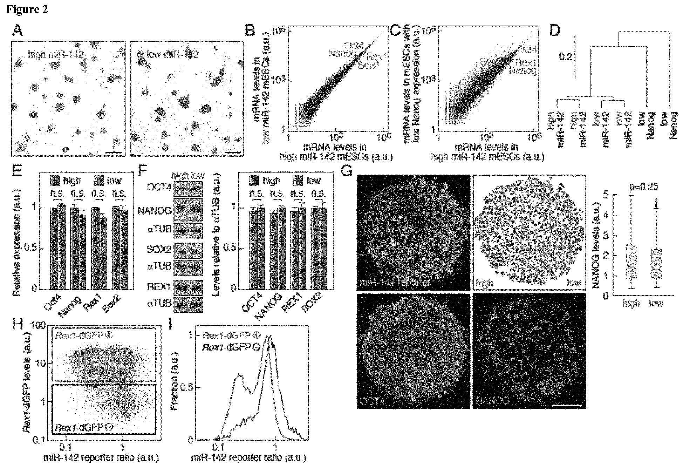

Figure 2 shows that "High" and "low" miR-142 cells express pluripotency markers at equal levels. (A) Alkaline phosphatase staining of FACS-purified "high" and "low" miR-142 state mESCs. Cells were cultured for 24 h after sorting and stained. Bar: 100 mm. (B) Deep sequencing analysis of mRNA expression levels in FACS-purified "high" and "low" miR-142 mESCs. (C) Deep sequencing analysis of mRNA expression levels in FACS-purified "high" miR-142 mESCs and mESCs with low Nanog expression. (D) Average linkage hierarchical clustering of mRNA profiles of "high" and "low" miR-142 mESCs and of mESCs with low Nanog expression. (E) mRNA expression levels of pluripotency markers in FACS-purified "high" and "low" miR-142 state mESCs (n=2; n.s.: not significant, two-sided t-test). (F) Western blot analysis and quantification of pluripotency marker levels in FACS-purified "high" and "low" miR-142 state mESCs (n=7; n.s.: not significant, two-sided t-test). (G) Immunostaining of OCT4 and NANOG in a clonal miR-142-3p reporter mESC colony. Quantification of NANOG levels in individual cells showed no significant difference in NANOG expression between the "high" and "low" miR-142 states (p=0.25, Kolmogorov-Smirnov test). As individual mESCs are not motile within a colony, sister lineages are spatially clustered. Bar: 100 mm. (H) miR-142 activity reporter ratio in a Rex1-dGFP knock-in mESC line. (I) Distribution of miR-142 reporter ratio in cells positive for Rex1-dGFP expression (Rex1-dGFP+, gray line) and negative for Rex1-dGFP expression (Rex1-dGFP-, black line). Gates identifying the populations are displayed in panel H.

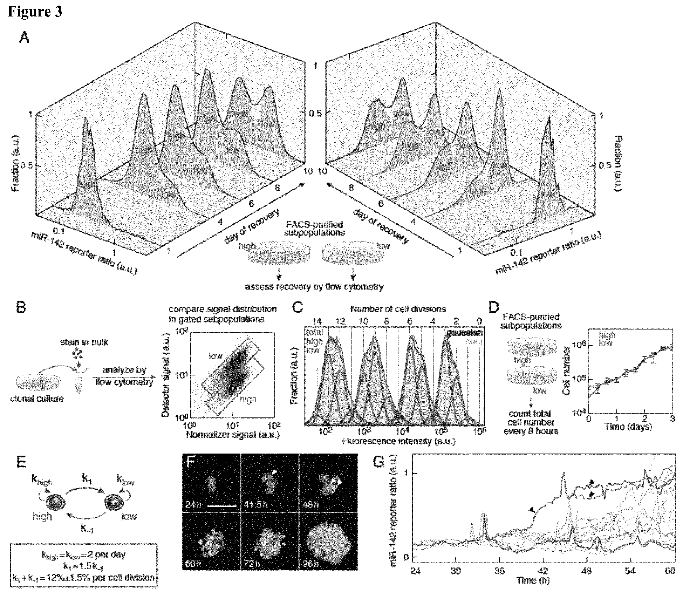

Figure 3 shows interconversion between the two miR-142 states. (A) "High" and "low" miR-142 subpopulations were FACS-purified and the temporal evolution of the miR-142 reporter ratio was measured. Shaded areas: quantification of cells in "high" or "low" miR-142 states. (B) Scheme of the experimental design to compare proliferation rates of mESCs in the "high" and "low" miR-142 state using a dye dilution by cell division strategy. (C) Time-lapse analysis of the fluorescence intensity of cells stained on day 0 with a commercial dye labeling free amines. The mESC culture was analyzed by flow cytometry each day. The distribution of the dye retention in all live cells in the culture (dark line) could be well approximated by the sum (shaded grey area) of gaussian distributions (white lines outlined in black) representing distinct cell division cycles. The distribution of dye retention in mESCs in the "high" or "low" miR-142 state is outlined by a gray or dark gray line. (D) Population growth of mESCs starting from FACS-purified "high" (gray line) and "low" (dark gray line) miR-142 subpopulations (error bars represent S.E.M., n=6). (E) Reaction kinetics model of the interconversion between "high" and "low" miR-142 cells. khigh and klow are the proliferation rates of "high" and "low" miR-142 cells, $k_1$ the interconversion rate from "high" to "low" miR-142 state and $k_{-1}$ the interconversion rate from "low" to "high" miR-142 state. (F) Live imaging of switching events during the growth of a single cell-derived mESC colony. Maximal projections of confocal stacks are shown at the indicated time points (h: hour). White arrowheads denote the cell with the first switching event at 41.5 h and the two resulting daughters at 48 h. Bar: 50 mm. (G) Single cell tracks of reporter ratio in two sister lineages (dark gray: sister lineage with activity switching, gray and light gray: sister lineage without activity switching, gray: all other lineages; spikes in reporter signal are artifacts due to signal saturation at mitotic divisions, black arrowheads correspond to the cells marked by white arrowheads in panel F).

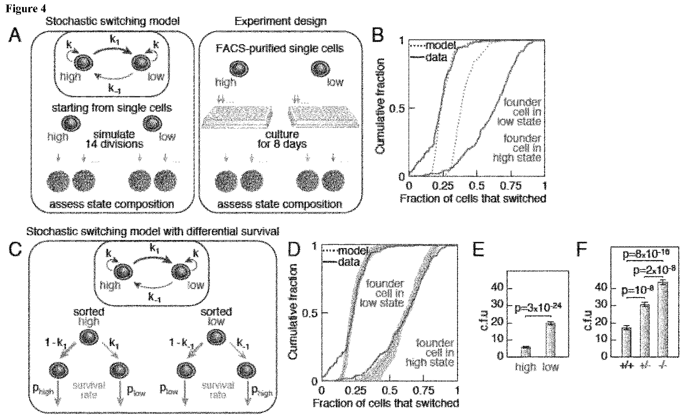

Figure 4 shows that mESCs switch stochastically between the two miR-142 states. (A) Stochastic switching model (left panel): cells can switch state each cell division with probability $k_1$ or $k_{-1}$. Experimental scheme (right panel): Clonal cultures were derived from single FACS-purified "high" and "low" miR-142 mESCs. Occurrence of switching events was measured by assessing the miR-142 reporter ratio distribution in individual cultures. (B) Simulation of state distribution after colony growth following the model shown in panel A with a founder cell in "low" miR-142 (dashed gray line) or "high" miR-142 (dashed light gray line) state (170 colonies, 14 divisions, $k_1 + k_{-1} = 0:08$ per cell division, $k_1 = 1:5 k_{-1}$). Solid light gray and gray lines: experimentally measured state distribution in cultures derived from FACS-purified founder cells in "high" and "low" miR-142 states (n=169 and n=171). (C) Stochastic switching model with differential survival. "High" and "low" miR-142 mESCs can have different survival rate under clonogenic conditions. (D) Simulation of state distribution after colony growth following the stochastic switching model with differential survival shown in panel C with a founder cell in "low" miR-142 (dotted dark gray line) or "high" miR-142 (dotted gray line) state (170 colonies, 14 divisions, $k_1 + k_{-1} = 0{:}08$ per cell division, $k_1 = 1{:}5$ $k_{-1}$, $p_{low} = p_{high} = 8$). Shaded area: 95% confidence interval. Solid lines: experimental data for FACS-purified founder cells in "high" and "low" miR-142 states, same data as shown in panel B. (E) Clonogenicity of single FACS-purified founder cells in "high" or "low" miR-142 state (c.f.u.: colony forming units; n=50; p=3 x $10^{-24}$, two-sided t-test; error bars represent S.E.M.). Single cells were FACS-purified in 96-well plates (n represents the number of 96-well plates that were analyzed). (F) Clonogenicity of single mir142+/+ (n=19), mir142+/- (n=20) or mir142-/- (n=20) mESCs (c.f.u.: colony forming units; p = $10^{-8}$, p = 2 x $10^{-8}$ and p = 8 x $10^{-16}$, two-sided t-test; error bars represent S.E.M.). Single cells were FACS-purified in 96-well plates (n represents the number of 96-well plates that were analyzed).

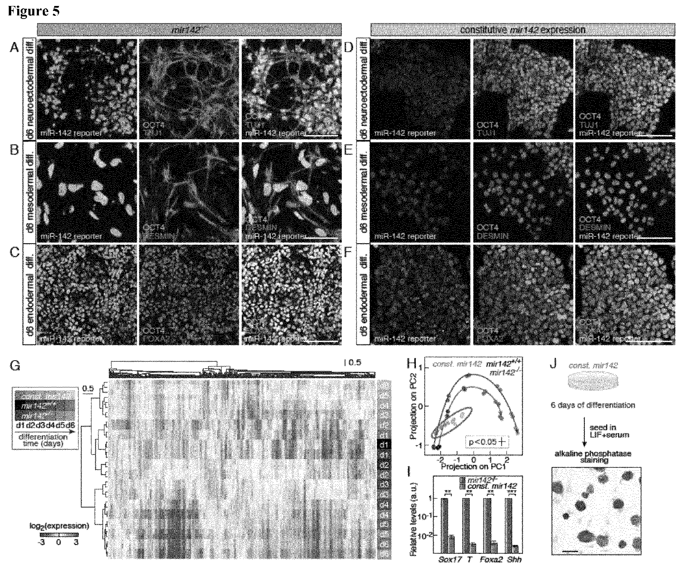

Figure 5 shows that mir142 expression locks mESCs in an undifferentiated state. (A) miR-142 reporter signal (left panel) and immunostaining of TUJ1 and OCT4 (middle panel) in mir142-/- mESCs differentiated for 6 days to neuroectoderm. Right panel: merge of left and middle panels. Bar: 100 mm. (B) miR-142 reporter signal (left panel) and immunostaining of DESMIN and OCT4 (middle panel) in mir142-/- mESCs differentiated for 6 days to mesoderm. Right panel: merge of left and middle panels. Bar: 100 mm. (C) miR-142 reporter signal (left panel) and immunostaining of FOXA2 and OCT4 (middle panel) in mir142-/- mESCs differentiated for 6 days to endoderm. Right panel: merge of left and middle panels. Bar: 100 mm. (D) miR-142 reporter signal (left panel) and immunostaining of TUJ1 and OCT4 (middle panel) in mESCs with constitutive mir142-expression differentiated for 6 days to neuroectoderm. Right panel: merge of left and middle panels. Bar: 100 mm. (E) miR-142 reporter signal (left panel) and immunostaining of DESMIN and OCT4 (middle panel) in mESCs with constitutive mir142-expression differentiated for 6 days to mesoderm. Right panel: merge of left and middle panels. Bar: 100 mm. (F) miR-142 reporter signal (left panel) and immunostaining of FOXA2 and OCT4 (middle panel) in mESCs with constitutive mir142-expression differentiated for 6 days to endoderm. Right panel: merge of left and middle panels. Bar: 100 mm. (G) Hierarchical clustering of mRNA expression in mir142+/+, mir142-/- mESCs or mESCs with constitutive mir142-expression (const. mir142) during differentiation to endoderm progenitors. (mir142+/+: black; mir142-/-: blue; const. mir142: orange; shading denotes the day of differentiation according to the boxed legend). (H) Principal component analysis of genome-wide mRNA expression during differentiation of mir142+/+ (black dots), mir142-/- (blue dots) mESCs and mESCs with constitutive mir142-expression (const. mir142, orange dots). PCI accounting for 60.6% of the variation was characterized by contributions of pluripotency and endoderm differentiation-associated genes, while PC2 (13.4% of the variation) was contributed by genes subjected to transient up- or down-regulation during the differentiation process. Shading denotes the day of differentiation according to the boxed legend in panel G. Black and dak gray arrows depict the differentiation trajectory of mir142+/+ and mir142-/- cells respectively. (I) Endoderm marker expression in mir142-/- (dark gray) cells and cells with constitutive mir142-expression (gray) differentiated for 6 days (n= 2, **: p<0:01, ***: p<0:001, two-sided t-test. Data represented as mean +/-S.E.M.). (J) mESCs with constitutive mir142-expression (const. mir142) were differentiated for 6 days, replated in pluripotency-maintaining conditions and stained for alkaline phosphatase. Bar: 100 mm.

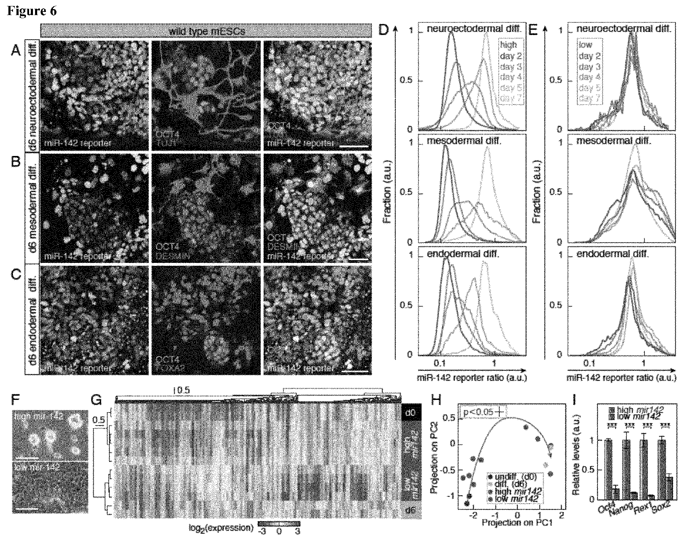

Figure 6 shows that the mir142-expressing subpopulation is delayed in differentiation. (A) miR-142 reporter signal (left panel) and immunostaining of TUJ1 and OCT4 (middle panel) in wild type mESCs differentiated for 6 days to neuroectoderm. Right panel: merge of left and middle panels. Bar: 50 mm. (B) miR-142 reporter signal (left panel) and immunostaining of DESMIN and OCT4 (middle panel) in wild type mESCs differentiated for 6 days to mesoderm. Right panel: merge of left and middle panels. Bar: 50 mm. (C) miR-142 reporter signal (left panel) and immunostaining of FOXA2 and OCT4 (middle panel) in wild type mESCs differentiated for 6 days to endoderm. Right panel: merge of left and middle panels. Bar: 50 mm. (D) Distribution of miR-142 activity reporter expression in FACS-purified "high" miR-142 cells differentiated to neuroectoderm, mesoderm and endoderm. Shading denotes the time course of differentiation according to the boxed legend in the top panel. (E) Distribution of miR-142 activity reporter expression in FACS-purified "low" miR-142 cells differentiated to neuroectoderm, mesoderm and endoderm. Shading denotes the time course of differentiation according to the boxed legend in the top panel. (F) Morphology of "high" and "low" miR-142 cells exposed for 3 days to endoderm differentiation cues. Bar: 100 mm. (G) Hierarchical clustering of mRNA expression of undifferentiated wild type mESCs (d0, black), wild type mESCs differentiated for 6 days (d6, gray) or subpopulations at day 3 of differentiation sorted according to their mir142 levels (light gray: high mir142; dark gray: low mir142). (H) Projection on the first two principle components PC1 and PC2 of mRNA expression profiles of high mir142 (red dots) and low mir142 (green dots) at day 3 of differentiation (black dots: undifferentiated mESCs at day 0; gray dots: differentiated cells at day 6). The black arrow represents the differentiation trajectory of wild type cells using data from Figure 5H. (I) Pluripotency marker expression in high mir142 and low mir142 cells at day 3 of differentiation (n=5 and n=4 respectively, ***: p < 0:001, two-sided t-test. Data represented as mean +/- S.D.).

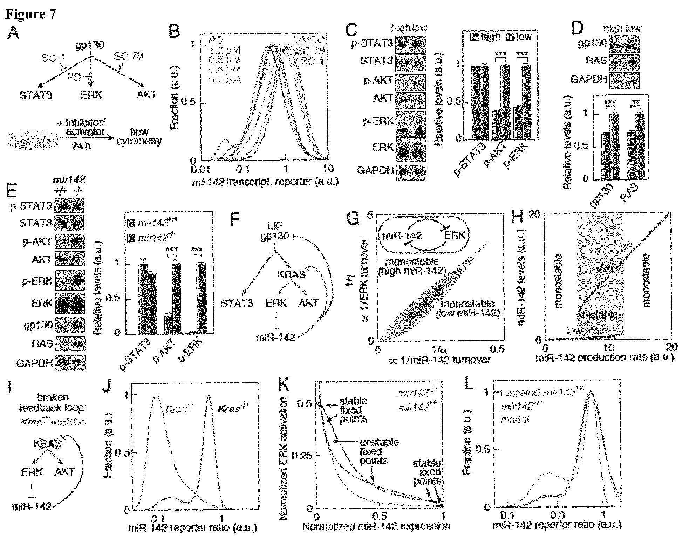

Figure 7 shows that Ras/ERK-signaling and miR-142 form a double-negative feedback loop and produce a bistable system. (A) Pharmacological interrogation of gp130-stimulated pathways. SC 79 activates AKT, SC-1 inhibits STAT3 activation and PD0325901 (PD) inhibits ERK activation. (B) Distribution mir142 transcriptional reporter expression under pharmacological interrogation of gp130-stimulated pathways (orange lines shaded according to the concentrations shown in the panel: PD; green line: SC 79; magenta line: SC-1; gray line: DMSO control). (C) Activation status of ERK, AKT, STAT3 and quantification in FACS-purified "high" and "low" miR-142 mESCs (n= 5, ***: p < 0:001, two-sided t-test. Data represented as mean +/- S.E.M.). (D) Protein levels of gp130 and RAS and quantification in FACS-purified "high" and "low" miR-142 mESCs (n=5; **: p < 0:01, ***: p < 0:001, two-sided t-test. Data represented as mean +/- S.E.M.). (E) Activation status of ERK, AKT, STAT3 and protein levels of gp130 and RAS in mir142+/+ and mir142-/- mESCs and quantification (n=4; ***: p < 0:001, two-sided t-test; error bars represent S.E.M.). (F) miR-142 and Ras/ERK signaling form a double-negative feedback loop. (G) Theoretical phase diagram of the miR-142-ERK double-negative feedback loop. a represents a rescaled miR-142 turnover and g an ERK activation turnover. Wild type mESCs sit in the bistability region (gray). (H) Simulated miR-142 levels depending on miR-142 production rate. At low production rates, there exists a single low miR-142 state. For intermediate production rates, two stable high and low miR-142 states coexist. Finally a single high miR-142 state is found at high miR-142 production rates. (I) Design of a system with a broken feedback loop through the deletion of Kras (Kras-/- mESCs). (J) Distribution of the miR-142 reporter ratio in Kras-/- mESCs (gray line) and wild type mESCs (Kras+/+, black line). (K) Nullclines of the miR-142 and ERK-signaling double-negative feedback loop (black line: nullcline corresponding to mir142+/+ cells; purple line: nullcline corresponding to mir142+/- cells). (L) Prediction of mir142 expression levels in mir142+/- mESCs. Using the calibration of the reporter ratio response shown in Figure 1G, the inventors could rescale the mir142+/+ distribution (gray line) to derive predicted values of miR-142 concentration in the two stable miR-142 states. The reporter ratio distribution in mir142+/- mESCs (gray line) could be well approximated by the model (light gray dotted line, fitting parameters: state occupancies).

SEQ ID NO: 1 shows the sequence of miR-142-5p (human). SEQ ID NO: 2 shows the sequence of miR-142-3p (human). SEQ ID NO: 3 shows the sequence of pre-miR-142 (human).

SEQ ID NO: 4 shows the sequence of the human miR-142 gene (NC_000017), with the complement (31..53)/product hsa-miR-142-3p, and the complement (69..89)/product "hsa-miR-142-5p".

SEQ ID NOs: 5 and 6 show the sequences of guide RNA inserts (mouse). SEQ ID NOs: 7 to 10 show the sequences of target sequences for Kras (mouse).

## EXAMPLES

**Construct design.** All constructs were cloned using the MXS-chaining approach (Sladitschek and Neveu, 2015).

**miRNA reporter constructs.** PCR-amplified DNA fragments encoding the open reading frame of H2BmCherry and H2B-Citrine were placed on either side of a bidirectional promoter. The PGK-promoter based version consisted of four PGK enhancer elements between two head-to-head oriented minimal PGK promoters (McBurney et al., 1991). The version based on the CAG-promoter (Niwa et al., 1991) consisted of four CMV immediate-early enhancer elements between two head-to-head arranged fragments containing the first exon and partial intron of chicken b-actin gene linked to the splice acceptor of the rabbit b-globin gene. The rabbit bGpA was used for both fluorescent proteins, but a binding site, perfectly complementary to the miRNA to be monitored was incorporated 11 bp downstream of the Citrine stop codon. A PGK::hygroRbGHpA cassette was included for selection. For use in the Rex1-dGFP knock-in mESC line (kindly provided by Austin Smith), the inventors constructed an activity reporter based on the same bidirectional CAG-promoter driving the expression of H2B-2xTagBFP as normalizer and H2B-Cherry as detector. Stable mESCs lines expressing the activity reporter were generated.

**mir142 transcriptional reporter.** The 1.52 kb fragment upstream of the mir142-hosting lin-cRNA gene (ENSMUSG00000084796) was PCR-amplified from mouse genomic DNA from 129 genetic background and cloned in front of H2B-TagBFPx3-bGpA. The plasmid contained a PGK::neoR-bGHpA cassette for selection. A stable transgenic mESC line expressing the mir142 transcriptional reporter was established in a line stably expressing the miR-142 activity reporter.

**Inducible mir142 construct.** The lincRNA containing gene (ENSMUSG00000084796) was PCR-amplified from mouse genomic DNA from 129 genetic background and cloned on one side of a bidirectional Tet-promoter. On the other side of the promoter, the inventors cloned NLS-TagBFPx3-PEST2D-bGHpA to quantify the induction level. The plasmid contained a PGK::neoR-bGHpA selection cassette and a PGK::rtTA-bGHpA cassette. A stable cell line (in a background stably expressing the miR-142 activity reporter) expressing mir142 in the presence of doxycycline at a dosage corresponding to 1.5 times the levels of endogenous mir142 expression in "high" miR-142 state cells was used as mir142 gain-of-function mESCs. Total miR-142 levels in those cells represented 0.05% of the total miRNA pool and were similar to the endogenous miR-142 expression levels found in the top 10% "high" miR-142 state cells, ruling out any overload of the miRNA biogenesis machinery. The mir142 stem loop-encoding fragment was replaced by a control miRNA stem loop in the control plasmid and a stable transgenic mESC line containing the construct was established in a line stably expressing the miR-142 activity reporter.

**Cell culture.** Mouse ESCs (R1 provided by the EMBL Transgenic Service or E14tga2, a kind gift of Michael Elowitz), were maintained without feeders in "LIF+serum" medium (DMEM high glucose, no glutamine, with sodium bicarbonate, Invitrogen) supplemented with 15% ES-qualified EmbryoMax Fetal Calf Serum (Millipore), 10 ng/ml murine LIF (EMBL Protein Expression and Purification Core Facility), 1x Non-Essential Amino Acids, 2 mM L-glutamine, 1 mM sodium pyruvate, 100 U/ml penicillin and 100 mg/ml streptomycin, 0.1 mM 2- mercaptoethanol (all Invitrogen) on culture dishes (Nunc) coated with 0.1% gelatin (Sigma) solution and cultured at 37_C with 5% CO2. N2B27 medium was prepared from a 1:1 mixture of DMEM/F12 (without HEPES, with L-glutamine) and neurobasal medium with 0.5x B-27 (without vitamin A) and 0.5x N-2 supplements, 100 U/ml penicillin and 100 mg/ml streptomycin, 0.25 mM L-glutamine, 0.1 mM 2-mercaptoethanol (all Invitrogen), 10 mg/ml BSA fraction V and 10 mg/ml human recombinant insulin (both Sigma). "2i" medium (Ying et al., 2008) was N2B27 medium supplemented with 3 mM CHIR99021 and 1 mM PD0325901 (both Tocris Bioscience). "LIF+BMP4" (Ying et al., 2003a) medium was prepared by adding 10 ng/ml murine BMP4 (R&D Systems) to N2B27 medium with 10 ng/ml murine LIF. Cells under primed pluripotency conditions (Greber et al., 2010) were cultured in N2B27 medium with 12 ng/ml murine FGF2 and 20 ng/ml murine Activin A (both Pepro-Tech). Medium was changed daily and cells were passaged every other day with 0.05% Trypsin-EDTA or StemPro Accutase (Invitrogen) at a passaging ratio of 1/3-1/12. mESCs were differentiated to endoderm precursors, mesoderm precursors and neuroectoderm precursors as described (Borowiak et al., 2009; Torres et al., 2012; Ying et al., 2003b).

**Generation of mir142+/-, mir142-/- mESCs and rescue of mir142 knock-out.** RNA-guided Cas9 nucleases were used to delete the mir142 gene. Two guide RNA inserts (with genome target sequences: 5'-GGTGGCCTGAAGAATCCCCG (SEQ ID NO: 5), 5'-GGAGCCATGAAGGTCTTTCG (SEQ ID NO: 6) were designed and cloned in pX330-U6-Chimeric-BB-CBh-hSpCas9 following Hsu et al. (2013). Both Cas9 plasmids were cotransfected in mESCs stably expressing the miR-142 activity reporter. Successfully edited clones corresponding to mir142+/- and subsequently to mir142-/- were identified by the derepression of the miR- 142-3p activity reporter and the deletion was confirmed by sequencing of genomic PCR products. For the mir142-/- rescue construct, the 1.52 kb upstream region and the mir142-hosting lincRNA gene (ENSMUSG00000084796) were combined and a PGK::puroR-bGHpA cassette was added for selection. Stable transgenic mESC lines expressing the construct were generated in a mir142-/-/miR-142 activity reporter background.

**Generation of Kras-/- mESCs.** RNA-guided Cas9 nucleases were used to delete the Kras gene. Four guide RNA inserts targeting the first exon of Kras which is essential (Johnson et al., 1997) (with genome target sequences: 5'-TATACTCAGTCATTTTCAGC (SEQ ID NO: 7), 5'-GACTGAGTATAAACTTGTGG (SEQ ID NO: 8), 5'-CTGAATTAGCTGTATCGTCA (SEQ ID NO: 9), 5'-GCTAATTCAGAATCACTTTG (SEQ ID NO: 10)) were designed and cloned in pX330-U6-Chimeric-BB-CBh-hSpCas9 following Hsu et al. (2013). The four Cas9 plasmids were cotransfected in mESCs stably expressing the miR-142 activity reporter. Successfully edited clones corresponding to Kras-/- were validated by checking for the deletion of the first exon by genomic PCR and the absence of any KRAS protein expression by Western blot.

**Flow cytometry and fluorescence-activated cell sorting.** Cells were analyzed on an LSR-Fortessa flow cytometer (BD BioSciences). FACS-purification was carried out using two MoFlo sorters (DakoCytomation) or a BD Influx sorter (BD BioSciences).

**Pharmacology.** mESCs were cultured in LIF+serum supplemented with the following compounds at the indicated concentrations: AKT activator SC 79 (Tocris) at 5 mM, MEK inhibitor PD0325901 (Tocris) at 0.2-1.2 mM, STAT3 inhibitor SC-1 (Sigma) at 5 mM and GSK-3 inhibitor CHIR99021 (Tocris) at 3 mM. DMSO served as vehicle and as negative control in all cases.

**RNA-seq library construction.** RNA was extracted from cells trypsinized from plates or pelleted directly after FACS-sorting using the Mir-Vana kit (Ambion) following the manufacturer's instructions. miRNA and mRNA libraries were prepared from the same total RNA sample. 21 barcoded miRNA libraries were prepared using NEBNext Multiplex Small RNA Library Prep Set for Illumina (New England Biolabs) following the manufacturer's instructions. 78 barcoded mRNA libraries were prepared using TruSeq RNA Sample Preparation (Illumina) following the manufacturer's instructions. Libraries were run on Illumina HiSeq 2000 in the 50SE regime.

**RNA-seq analysis:** The inventors built a Bowtie index for Ensembl cDNAs of the mouse genome release GRCm38 masked with Repeat-Masker (Smit, AFA, Hubley, R and Green, P. RepeatMasker Open-3.0. 1996-2010 http://www.repeatmasker.org). mRNA reads were aligned to this index using Bowtie (Langmead et al., 2009) with default parameters. mRNA read counts were determined for each Ensembl ID using custom Python scripts. Read counts were not normalized by the transcript length for individual genes as we were solely interested in relative expression changes across samples. After trimming, miRNA reads were matched to miRBase release 19 (Griffiths-Jones et al., 2008) mouse sequences allowing no mismatch using custom Python scripts. Read counts were normalized to account for different sequencing depth. The normalization factor was determined by matching median-filtered log-transformed read counts for two samples to the identity line. For hierarchical clustering analysis, we kept genes with a maximal expression >1 transcript per cell across samples and at least a 4-fold variation in expression and used Pearson's correlation coefficient as a distance. Principle component analysis was carried out as described in Neveu et al. (2010).

**Live imaging.** mESCs were seeded at single-cell density (200 cells/cm$^2$) onto gelatin-coated Lab-Tek glass-bottom chamber slides (Nunc) or m-slides (Ibidi). Confocal sections were acquired on an inverted SP8 confocal microscope (Leica) equipped with 40x PL Apo 1.1 W objective in an incubation chamber at 37°C under a humidified 5% CO$_2$ atmosphere. Citrine and mCherry were excited with 514-nm and 561-nm lasers, and green and red signals were acquired sequentially using HyD detectors. Images were segmented using custom Python scripts. Phase contrast bright-field images were acquired on an inverted DM IL LED (Leica) microscope with HI PLAN I 10x/0.22 PH1 and HI PLAN I 20x/0.30 PH1 objectives.

**Modeling of the activity reporter response.** The inventors modeled the reporter ratio r dependence on the miRNA concentration M by r = 1=(1+Mn=Kn) where K is the binding constant and n is the Hill coefficient of the interaction. Using deep sequencing results for the reporter transcript and miR-142-3p from the same sample, the inventors found that n = 1:07_0:04, i.e. there is no cooperativity in the reporter response.

**Modeling of state switching**

**Population behavior.** The temporal dynamics of the interconvertible system of the "high" and "low" miR-142 states is governed by the following equations:

$$\frac{dH}{dt} = k_{high}H - k_1 H + k_{-1} L$$

$$\frac{dL}{dt} = k_{low}L + k_1 H - k_{-1} L$$

where H and $L$ are the number of cells in the "high" and "low" miR-142 states respectively, $k_{high}$ and $k_{low}$ are the division rates of the two states, $k_1$ the switching rate from "high" to "low" miR-142 state and $k_{-1}$ the switching rate from "low" to "high" miR-142 state. Given $k_{high} = k_{low} = k$ from experimental data, the evolution of the population reads:

$$H = \left( \frac{k_{-1}}{k_1 + k_{-1}} (H_0 + L_0)(1 - e^{-(k_1 + k_{-1})t}) + H_0 e^{-(k_1 + k_{-1})t} \right) e^{kt}$$

where $H_0$ and $L_0$ are the initial number of cells in "high" and "low" miR-142 state.

**Single-cell behavior.** To model culture-reconstitution experiments from single founder cells, the inventors allowed for stochastic state switching once per cell cycle with probability $k_1$ and $k_{-1}$ for 14 divisions (corresponding roughly to the 15,000-strong cell population analyzed in the experiment) using $k_1 = 1:5\, k_{-1}$. The inventors introduced a survival bias for "low" miR-142 cells compared to "high" miR-142 cells under single-cell plating conditions for the first two cell divisions. Two parameters were adjusted: $k_1 + k_{-1} = 0:08$ per cell division and the survival bias which was set to 8. State distribution was determined for 170 independently simulated colonies. Confidence intervals were determined by simulating 100 times 170 colonies for each miR-142 state.

**Modeling of the miR-142-ERK signaling double-negative feedback loop.** The temporal dynamics of the double-negative feedback loop between miR-142 and LIF-induced ERK signaling was modeled by the following equations:

$$\frac{dM}{dt} = -k_{d1}M + k_1 \frac{1}{1 + E^{n_E}/K_E^{n_E}}$$

$$\frac{dE}{dt} = -k_{d2}E + k_2 \frac{1}{1 + M^{n_1}/K_1^{n_1}}(E^{tot} - E)$$

where M is the concentration of miR-142, $E$ the fraction of active ERK, $k_{d1}$ and $k_{d2}$ the degradation rate of miR-142 and active ERK, $E^{tot}$ the total concentration of ERK (assumed to be constant), $K_E$ the active ERK repression constant, $k_1$ the production rate of miR-142, $k_2$ the maximum activation rate of ERK, $n_E$ the Hill coefficient of ERK-mediated miR-142 repression and $n_1$ the Hill coefficient of the miR-142-mediated ERK repression. ERK is known to dimerize (Khokhlatchev et al., 1998) so $n_E = 2$. miR-142 represses multiple components of the LIF-induced MEK/ERK cascade so $n_1 > 1$. He inventors took $n_1 = 2$ without any loss of generality of the findings. By introducing the parameters $\alpha = k_1/K_1 k_{d1}$, $\beta = E^{tot}/K_E$, $\gamma = k_2/k_{d2}$, and introducing the rescaled quantities $X = M/\alpha K_1$ and $Y = E/E^{tot}$, the inventors can rewrite the system as a function of X and Y:

$$\frac{dX}{dt} = -k_{d1}X + \frac{k_{d1}}{1+\beta^2 Y^2}$$

$$\frac{dY}{dt} = -k_{d2}Y + \frac{k_2}{1+\alpha^2 X^2}(1-Y)$$

$\alpha$ represents a rescaled miR-142 turnover and $\gamma$ an ERK activation turnover. The nullclines are:

$$Y = \frac{\gamma}{1+\gamma+\alpha^2 X^2}$$

$$Y = \frac{1}{\beta}\sqrt{\frac{1}{X}-1}$$

mir142+/- cells are equivalent to having a miR-142 production rate of $k_1/2$. The inventors determined numerically steady state solutions in the $\alpha$, $\beta$ and $\gamma$ parameter space. Theoretical phase diagram (Figure 7G) is shown for b = 10. miR-142 expression levels (Figure 7H) are computed for $0 \leq \alpha \leq 20$, $\beta = 10$, $\gamma = 1$. Nullclines (Figure 7K) are computed for $\alpha = 12$, $\beta = 10$, $\gamma=1$.

**Statistical analysis.** Statistical tests were computed using the Python SciPy module. When appropriate, the inventors corrected for multiple hypothesis testing following Benjamini and Hochberg (1995).

**Primary data.** Sladitschek HL, Neveu PA (2014). A toggle-switch between miR-142 and LIF-signaling creates heterogeneity among mouse ES cells. ArrayExpress E-MTAB-2830, E-MTAB-2831 and E-MTAB-3234.

**Results**

**miR-142 is a new marker of mESC heterogeneity under naïve pluripotency conditions**

The inventors reasoned that miRNAs that control different self-renewing mESC states should show heterogeneous expression under uniform pluripotency-maintaining conditions. To identify such miRNAs, the inventors devised a ratiometric fluorescence sensor that can visualize miRNA activity in single cells. The reporter consists of a bidirectional promoter driving the expression of a normalizer (H2B-mCherry) and a miRNA detector (H2BCitrine), which contains in its 3'-UTR a target sequence of the miRNA of interest (Figures 1A).

Using this reporter system, the inventors screened 33 conserved miRNAs associated with differentiation, pluripotency or cell proliferation in mESC lines stably expressing specific reporters. As expected, the inventors found the abundant miR-294 to be highly active, whereas the differentiation-associated miRNA let-7 showed little activity. Most miRNA reporters displayed a normally distributed cell-tocell variation comparable to a non-targeted control. Strikingly, however, the inventors found a strongly variegated activity of miR-142-3p that divided clonal mESC colonies into two sectors with very different miRNA activity (Figure 1B). Also at the population level, miR-142-3p activity was clearly bimodal distinguishing two mESC populations with either a high or a low miR-142-3p activity state (in the following referred to as "high" and "low" miR-142 states, Figures 1C and D). Furthermore, this bimodal regulation was present in chemically defined media conditions that support naive pluripotency including "2i" but was absent in primed pluripotency. Thus, the bimodal regulation of mir-142 represents a novel kind of mESC heterogeneity in LIF-dependent pluripotency.

To validate that the inventors' reporter responded specifically to miR-142-3p, the inventors generated mir142-/- mESC lines by deleting both alleles of mir142 using the CRISPR/Cas9 technology. As expected, the repression of the reporter was relieved in mir142-/- cells (Figure 1E). In addition, the inventors assessed miRNA expression levels of FACS-purified "high" and "low" miR-142 state subpopulations in wild type mESCs by deep sequencing.

This analysis showed a 10-fold increase in the expression levels of miR-142-3p and miR-142-5p, the two mature forms of the miR-142 stem loop, in "high" miR-142 mESCs compared to "low" miR-142 mESCs (Figure 1F). Expression levels of all the other detected miRNAs were tightly correlated between the "high" and "low" miR-142 states (Figure 1F). Finally, the inventors calibrated our single-cell miRNA activity reporter using expression data of miR-142-3p and mRNA reporter levels measured by deep-sequencing. As expected, the signal of miR-142 activity reporter depends on miR-142-3p levels following Hill's equation with noncooperative binding (Figure 1G). The inventors' reporter thus specifically measures the activity of miR-142-3p. Low miR-142-3p levels correspond to large reporter ratios and high miR-142-3p levels yield small reporter ratios. The system therefore allows us to quantitate miR-142 expression changes in single living cells.

**The two miR-142 states are indistinguishable by pluripotency markers.** Previous reports of mESC heterogeneity found a metastable coexistence of a pluripotent state and a state prone to differentiate and already expressing lower levels of the pluripotency markers Nanog and Rex1 (Chambers et al., 2007; Singh et al., 2007; Toyooka et al., 2008; Singer et al., 2014). To test if miR-142 heterogeneity is upstream of expression changes in pluripotency markers, the inventors analyzed FACS-purified "high" and "low" miR-142 mESC populations. The two miR-142 states were both positive for alkaline phosphatase staining (Figure 2A). mRNA profiles measured by deep sequencing of FACS-purified "high" and "low" miR-142 state mESCs clustered together, while the mRNA profiles of mESCs with low Nanog expression clustered apart (Figures 2B-D). Closer examination of pluripotency factor expression showed no significant difference in the mRNA or protein expression levels of Oct4 (or Pou5f1), Nanog, Rex1 (or Zfp42) and Sox2 between the "high" and "low" miR-142 states in FACS-purified subpopulations (Figures 2E and F). Furthermore, the "high" and "low" miR-142 states showed no difference in the known heterogeneity of NANOG (Figures 2G) or REX1 protein expression at the single cell level. Moreover, all cells stained positive for the pluripotency markers OCT4 and SSEA-1 irrespective of their "high" or "low" miR-142 state identities. In addition, neither "high" nor "low" miR-142 state cells shared molecular markers with epiblast stem cells, that reside in a state of primed pluripotency. Thus, the "high" and "low" miR-142 states are indistinguishable in their pluripotency marker expression and did not represent a primed pluripotent state.

Finally, the inventors introduced the miR-142 activity reporter in a Rex1-dGFP knock-in mESC line (Wray et al., 2011) in order to compare the bimodal regulation of miR-142 to the known heterogeneity in Rex1 expression. mESCs with high Rex1-dGFP levels revealed a bimodal regulation of miR-142 activity, i.e. mESCs with high Rex1-dGFP reside in either the "high" miR-142 state or the "low" miR-142 state (Figures 2H and I). Moreover, cells with low Rex1-dGFP expression had a unimodal reporter distribution with a reporter ratio comparable to mir142-/- mESCs, corresponding to an absence of miR-142 expression (Figures 2H and I). Thus, the "high" miR-142 state and the "low" miR-142 sate are only found in the high Rex1 mESC compartment.

This finding places miR-142 bimodality upstream of the so far described heterogeneity in pluripotency transcription factor expression. Therefore, miR-142 bimodality represents a novel kind of heterogeneity in naive mESCs.

**The two miR-142 states interconvert stochastically:** To assess if and how the two miR-142 states can interconvert into each other, the inventors monitored the distribution of miR-142 activity after FACS-purification of "high" or "low" miR-142 subpopulations (Figure 3A). Indeed either state could regenerate the other within 10 days of culture under pluripotency conditions (Figures 3A). State recovery was not due to any differential growth between the two miR-142 states because "high" and "low" miR-142 cells divided at the same rate every 12 h (Figures 3B-D). To determine the interconversion rates, the inventors quantified the fraction of the population in the "high" and "low" miR-142 states. The inventors then fitted the population data using first order reaction kinetics (Figures 3E). Cells converted from "high" to "low" miR-142 states with a rate $k_1 = 0.072$ +/- 0.01 per cell division (on average one switching event every 14 divisions), while the backconversion was slightly slower occurring with a rate $k_{-1} = 0.048$ +/- 0.006 per cell division (on average one switching event every 23 divisions). Investigating the reporter ratio distribution in cultures derived from FACS-purified single cells showed that cultures recovered both states whether starting from "high" or "low" miR-142 founder cells.

This demonstrated that all clonogenic cells can switch between states. Single-cell live imaging of miR-142 activity revealed that switching occurred rapidly within less than a cell cycle and that after division sister lineages were not always correlated in their switching behavior (Figures 3F, G) suggesting stochastic switching events.

If switching were indeed stochastic, the variegated distribution of "high"/"low" miR-142 cells in a colony grown from a single cell will depend on the time when the first state switching occurred, since the states are on average stable for several cell cycles (Figure 4A). Using a stochastic switching model, the inventors could simulate the expected fraction of cells that switched state in colonies derived from pure "high" or "low" miR-142 state single founders (Figure 4B). To test this prediction, the inventors measured the fraction of switched cells in colonies grown from single FACS-purified "high" or "low" miR-142 cells. The stochastic switching model approximated well data from founder cells FACS-purified in the "low" miR-142 state but could not fit with the same parameters the state composition obtained in cultures derived from founder cells FACS-purified in the "high" miR-142 state (Figure 4B). The inventors thus introduced a refined model in which cells stochastically switch between the two miR-142 states but "high" and "low" miR-142 cells can have different survival rates under clonogenic conditions while having the same proliferation rate (Figure 4C). The experimental data was recapitulated by simulations using this refined model including a survival bias for "low" miR-142 cells under clonogenic conditions (Figure 4D). Interestingly, the inventors experimentally confirmed this survival bias predicted by the model. Indeed, clonogenicity of FACS-purified "low" miR-142 cells (19.8 +/- 6.6%) was higher than for "high" miR-142 cells (5.8 +/- 3.1%) (Figure 4E). This gave a survival bias for "low" miR-142 cells of 6.9 (90% confidence interval: 1.8-17.8), in excellent agreement with the 8-fold survival bias predicted by the simulations. Using a genetic loss-of-function approach, the inventors could show that the loss of mir142 expression indeed improved clonogenicity without affecting the proliferation rate (Figure 4F). In summary, the inventors could demonstrate experimentally and theoretically that individual mESCs fluctuate stochastically between the two miR-142 states at a relatively low rate with a state switching event occurring on average every 8 cell divisions.

**Constitutive miR-142 expression locks cells in an undifferentiated state.** A hallmark of embryonic stem cells is the ability to generate distinct differentiated cell types. To assess whether mir142 expression affects differentiation capacity, the inventors compared mir142 gain- or loss-of-function mESCs regarding their capabilities to differentiate towards fates of the three germ layers, i.e. neuroectoderm, mesoderm and endoderm fate. Upon differentiation, mir142-/- cells stained positive for the neuronal marker Tuj1 (or bIII-tubulin), the muscle marker Desmin or the endoderm marker Foxa2 and were negative for the pluripotency marker Oct4 (Figures 5A-C). By contrast, mir142 gain-of-function cells retained Oct4 expression and showed no differentiation marker expression (Figures 5D-F). In order to understand genome-wide this striking difference in response to differentiation cues, the inventors profiled the transcriptomes of wild type mESCs, mir142-/- and mir142-expressing mESCs during a six day endoderm differentiation time course. Strikingly, cells constitutively expressing mir142 always clustered with undifferentiated wild type and mir142-/- cells at day 1 or 2 (Figure 5G), while differentiating wild type mESCs and mir142-/- cells from day 3-6 cluster separately. Using principle component analysis allowed the inventors to visualize the trajectory of expression profiles during six days of differentiation (Figure 5H). This showed that unlike wild type and mir142-/- cells, cells constitutively expressing mir142 were essentially locked in an undifferentiated expression state (Figures 5H) and consistently failed to up-regulate established endoderm markers (Figure 5I). Even at the end of the six day differentiation procedure, cells with constitutive mir142 expression proliferated normally under pluripotency conditions, exhibited the characteristic 3-dimensional morphology of undifferentiated mESC colonies and were alkaline phosphatase-positive (Figure 5J). In addition, genetic deletion of mir142 led to significantly larger changes in gene expression compared to wild type cells as measured by projection on PC1 and PC2. Indeed, mir142-/- cells exhibited significantly higher levels of differentiation markers and a lower expression of pluripotency markers compared to wild type cells at day 6 of differentiation. Together, the inventors' data demonstrates that mir142 expression locks mESCs in an undifferentiated state even if exposed to strong differentiation cues for several days.

**The "high" mir142 subpopulation is delayed in differentiation.** To test if the naturally generated "high" miR-142 state also locks cells in an undifferentiated state, the inventors differentiated wild type mESCs expressing the miR-142 reporter towards neuroectoderm, mesoderm and endoderm fate. Upon differentiation towards neuroectoderm, mesoderm and endoderm fate, cells with "low" miR-142 activity stained positive for the neuronal marker Tuj1 (or bIII-tubulin), the muscle marker Desmin or the endoderm marker Foxa2, respectively (Figures 6A-C). In contrast, cells exhibiting "high" miR-142 activity stained positive for the pluripotency marker OCT4 independently of the differentiation regime (Figures 6A-C). The invoentors next aimed to characterize the effect of the endogenous bimodal miR-142 expression during the differentiation of a wild type mESC population in more details. The inventors first monitored the changes of miR-142 activity in FACS-purified "high" or "low" miR-142 cell populations undergoing differentiation to neuroectoderm, mesoderm and endoderm. "High" miR-142 cells gradually lost miR-142 activity (and mir142 expression) over the first 4 days of differentiation irrespective of the differentiation regime, becoming in majority converted into a "low" miR-142 state by day 7 (Figures 6D). In contrast, "low" miR-142 cells did not change their miR-142 activity state under any differentiation cue (Figure 6E). Inspection of the differentiating cultures revealed that "high" miR-142 cells kept their 3-dimensional morphology up to 4 days, whereas "low" miR-142 cells readily adapted a differentiated monolayer morphology within 3 days (Figure 6F).

The inventors next aimed to gain a genome-wide view of the differences in gene expression in differentiating mESCs depending on their mir142 levels. To do so, they subjected a bimodal mESC population grown under pluripotency conditions to differentiation cues for 3 days, FACS-purified cell populations with either high or low mir142 expression and assessed gene expression by transcriptome profiling. Cells that exhibited high mir142 levels after three days of differentiation clustered with undifferentiated mESCs, whereas cells with no mir142 expression clustered with differentiated cells (Figure 6G). Projection onto the differentiation gene expression trajectory of wild type mESCs confirmed that mir142-expressing cells remained at the beginning of the differentiation trajectory, while the profiles of mir142-negative cells had progressed to the end of the trajectory similar to differentiated cells (Figure 6H). Probing specific genes showed that mir142-expressing cells failed to down-regulate pluripotency genes in response to instructive differentiation cues (Figures 6I).

To conclude, the naturally generated "high" miR-142 state locked cells in an undifferentiated state and the bimodal regulation of mir142 establishes a dichotomy between a subpopulation amenable to differentiation (the "low" miR-142 cells) and a pool of cells delayed in differentiation (the "high" miR-142 cells).

**miR-142 states differ in AKT and ERK activation.** Next, the inventors sought a mechanistic understanding of the bimodal expression of miR-142 under LIF-dependent pluripotency conditions. Deep sequencing and a single-cell mir142 transcriptional reporter demonstrated that transcriptional regulation accounted for the bimodal regulation of miR-142 activity. LIF is known to activate three distinct signaling pathways through its coreceptor gp130 (Figure 7A): the JAK/STAT3 (Niwa et al., 1998) and PI3K/AKT (Paling et al., 2004) pathways and the MEK/ERK signaling cascade (Burdon et al., 1999). Inhibiting ERK activity increased the expression of the mir142 transcriptional reporter in a dose-dependent manner, while inhibition of STAT3 or activation of AKT had no effect (Figures 7B). This indicated that ERK activity normally represses mir142 expression, predicting that cells with "high" miR-142 activity have reduced ERK activity. To test this, the inventors measured phosphorylated ERK kinase in FACS-purified "high" and "low" miR-142 state subpopulations. "High" miR-142 cells indeed showed reduced level of active ERK kinase compared to "low" miR-142 mESCs, while total ERK levels were unaffected ($p = 7 \times 10^{-7}$ for p-ERK, n = 5, two-sided t-test, Figure 7C). In addition phosphorylated AKT levels were reduced in "high" miR-142 cells compared to "low" miR-142 mESCs, while total AKT levels were unaffected as was phosphorylated and total STAT3 ($p = 8 \times 10^{-9}$ and $p = 0.78$ for p-AKT and p-STAT3, n = 5, two-sided t-test, Figure 7C). Thus, the miR-142 states correspond to two subpopulations of mESCs that differed in the activation status of ERK and AKT signaling.

**References**

Bartel, D. P. (2009). MicroRNAs: target recognition and regulatory functions. Cell, 136, 215-233

Benjamini, Y. and Hochberg, Y. (1995). Controlling the false discovery rate: a practical and powerful approach to multiple testing. J. R. Stat. Soc. B, 57, 289-300.

Blanpain, C. and Fuchs, E. (2014). Stem cell plasticity. Plasticity of epithelial stem cells in tissue regeneration. Science, 344, 1242281.

Borowiak, M., Maehr, R., Chen, S., Chen, A. E., Tang, W., Fox, J. L., Schreiber, S. L., and Melton, D. A. (2009). Small molecules efficiently direct endodermal differentiation of mouse and human embryonic stem cells. Cell Stem Cell, 4, 348-358.

Burdon, T., Stracey, C., Chambers, I., Nichols, J., and Smith, A. (1999). Suppression of SHP-2 and ERK signalling promotes self-renewal of mouse embryonic stem cells. Dev. Biol., 210, 30-43.

Canham, M. A., Sharov, A. A., Ko, M. S. H., and Brickman, J. M. (2010). Functional heterogeneity of embryonic stem cells revealed through translational amplification of an early endodermal transcript. PLoS Biol., 8, e1000379.

Chambers, I., Silva, J., Colby, D., Nichols, J., Nijmeijer, B., Robertson, M., Vrana, J., Jones, K., Grotewold, L., and Smith, A. (2007). Nanog safeguards pluripotency and mediates germline development. Nature, 450, 1230-1234.

Chapnik, E., Rivkin, N., Mildner, A., Beck, G., Pasvolsky, R., Metzl-Raz, E., Birger, Y., Amir, G., Tirosh, I., Porat, Z., et al. (2014). miR-142 orchestrates a network of actin cytoskeleton regulators during megakaryopoiesis. eLife, 3, e01964.

Chen, C.-Z., Li, L., Lodish, H. F., and Bartel, D. P. (2004). MicroRNAs modulate hematopoietic lineage differentiation. Science, 303, 83-86.

Cohen, D. E. and Melton, D. (2011). Turning straw into gold: directing cell fate for regenerative medicine. Nat. Rev. Genet., 12, 243-252.

Evans, M. J. and Kaufman, M. H. (1981). Establishment in culture of pluripotential cells from mouse embryos. Nature, 292, 154-156.

Foronda, D., Weng, R., Verma, P., Chen, Y.-W., and Cohen, S. M. (2014). Coordination of insulin and Notch pathway activities by microRNA miR-305 mediates adaptive homeostasis in the intestinal stem cells of the Drosophila gut. Genes Dev, 28, 2421-2431.

Greber, B., Wu, G., Bernemann, C., Joo, J. Y., Han, D. W., Ko, K., Tapia, N., Sabour, D., Sterneckert, J., Tesar, P., et al. (2010). Conserved and divergent roles of FGF signaling in mouse epiblast stem cells and human embryonic stem cells. Cell Stem Cell, 6, 215-226.

Griffiths-Jones, S., Saini, H. K., Van Dongen, S., and Enright, A. J. (2008). miRBase: tools for microRNA genomics. Nucleic Acids Res., 36, D154-8.

Grimson, A., Farh, K. K.-H., Johnston, W. K., Garrett-Engele, P., Lim, L. P., and Bartel, D. P. (2007). MicroRNA targeting specificity in mammals: determinants beyond seed pairing. Mol. Cell, 27, 91-105.

Gupta, P. B., Fillmore, C. M., Jiang, G., Shapira, S. D., Tao, K., Kuperwasser, C., and Lander, E. S. (2011). Stochastic state transitions give rise to phenotypic equilibrium in populations of cancer cells. Cell, 146, 633-644.

Hsu, P. D., Scott, D. A., Weinstein, J. A., Ran, F. A., Konermann, S., Agarwala, V., Li, Y., Fine, E. J., Wu, X., Shalem, O., et al. (2013). DNA targeting specificity of RNA-guided Cas9 nucleases. Nat. Biotechnol., 31, 827-832.

Jiang, H., Patel, P. H., Kohlmaier, A., Grenley, M. O., McEwen, D. G., and Edgar, B. A. (2009). Cytokine/Jak/Stat signaling mediates regeneration and homeostasis in the Drosophila midgut. Cell, 137, 1343-1355.

Johnson, L., Greenbaum, D., Cichowski, K., Mercer, K., Murphy, E., Schmitt, E., Bronson, R., Umanoff, H., Edelmann, W., Kucherlapati, R., et al. (1997). K-ras is an essential gene in the mouse with partial functional overlap with n-ras. Genes & Development, 11, 2468-2481.

Johnston, R. J., Chang, S., Etchberger, J. F., Ortiz, C. O., and Hobert, O. (2005). MicroRNAs acting in a double-negative feedback loop to control a neuronal cell fate decision. Proc. Natl Acad. Sci. USA, 102, 12449-12454.

Khokhlatchev, A. V., Canagarajah, B., Wilsbacher, J., Robinson, M., Atkinson, M., Gold-smith, E., and Cobb, M. H. (1998). Phosphorylation of the MAP kinase ERK2 promotes its homodimerization and nuclear translocation. Cell, 93, 605-615.

Klein, A. M., Nakagawa, T., Ichikawa, R., Yoshida, S., and Simons, B. D. (2010). Mouse germ line stem cells undergo rapid and stochastic turnover. Cell Stem Cell, 7, 214-224.

Kramer, N. J., Wang, W.-L., Reyes, E. Y., Kumar, B., Chen, C.-C., Ramakrishna, C., Cantin, E. M., Vonderfecht, S. L., Taganov, K. D., Chau, N., et al. (2015). Altered lymphopoiesis and immunodeficiency in miR-142 null mice. Blood, 125, 3720-3730.

Kueh, H. Y., Champhekar, A., Champhekhar, A., Nutt, S. L., Elowitz, M. B., and Rothenberg, E. V. (2013). Positive feedback between PU.1 and the cell cycle controls myeloid differentiation. Science, 341, 670-673.

Kunath, T., Saba-El-Leil, M. K., Almousailleakh, M., Wray, J., Meloche, S., and Smith, A. (2007). FGF stimulation of the Erk1/2 signalling cascade triggers transition of pluripotent embryonic stem cells from self-renewal to lineage commitment. Development, 134, 2895-2902.

Langmead, B., Trapnell, C., Pop, M., and Salzberg, S. L. (2009). Ultrafast and memory-efficient alignment of short DNA sequences to the human genome. Genome Biol., 10, R25.

Li, X. and Carthew, R. W. (2005). A microRNA mediates EGF receptor signaling and promotes photoreceptor differentiation in the Drosophila eye. Cell, 123, 1267-1277.

Losick, R. and Desplan, C. (2008). Stochasticity and cell fate. Science, 320, 65-68.

Martin, G. R. (1981). Isolation of a pluripotent cell line from early mouse embryos cultured in medium conditioned by teratocarcinoma stem cells. Proc. Natl. Acad. Sci. USA, 78, 7634-7638.

McBurney, M. W., Sutherland, L. C., Adra, C. N., Leclair, B., Rudnicki, M. A., and Jardine, K. (1991). The mouse Pgk-1 gene promoter contains an upstream activator sequence. Nucleic Acids Res., 19, 5755-5761.

Medema, J. P. and Vermeulen, L. (2011). Microenvironmental regulation of stem cells in intestinal homeostasis and cancer. Nature, 474, 318-326.

Mildner, A., Chapnik, E., Manor, O., Yona, S., Kim, K.-W., Aychek, T., Varol, D., Beck, G., Itzhaki, Z. B., Feldmesser, E., et al. (2013). Mononuclear phagocyte miRNome analysis identifies miR-142 as critical regulator of murine dendritic cell homeostasis. Blood, 121, 1016-1027.

Murry, C. E. and Keller, G. (2008). Differentiation of embryonic stem cells to clinically relevant populations: lessons from embryonic development. Cell, 132, 661-680.

Neumüller, R. A., Betschinger, J., Fischer, A., Bushati, N., Poernbacher, I., Mechtler, K., Cohen, S. M., and Knoblich, J. A. (2008). Mei-P26 regulates microRNAs and cell growth in the Drosophila ovarian stem cell lineage. Nature, 454, 241-245.

Neveu, P., Kye, M. J., Qi, S., Buchholz, D. E., Clegg, D. O., Sahin, M., Park, I.-H., Kim, K.-S., Daley, G. Q., Kornblum, H. I., et al. (2010). MicroRNA profiling reveals two distinct p53-related human pluripotent stem cell states. Cell Stem Cell, 7, 671-681.

Nimmo, R., Ciau-Uitz, A., Ruiz-Herguido, C., Soneji, S., Bigas, A., Patient, R., and Enver, T. (2013). miR-142-3p controls the specification of definitive hemangioblasts during ontogeny. Dev. Cell, 26, 237-249.

Niwa, H., Burdon, T., Chambers, I., and Smith, A. (1998). Self-renewal of pluripotent embryonic stem cells is mediated via activation of STAT3. Genes Dev., 12, 2048-2060.

Niwa, H., Yamamura, K., and Miyazaki, J. (1991). Efficient selection for high-expression transfectants with a novel eukaryotic vector. Gene, 108, 193-199.

North, T. E., Goessling, W., Walkley, C. R., Lengerke, C., Kopani, K. R., Lord, A. M., Weber, G. J., Bowman, T. V., Jang, I. H., Grosser, T., et al. (2007). Prostaglandin E2 regulates vertebrate haematopoietic stem cell homeostasis. Nature, 447, 1007-1011.

Paling, N. R. D., Wheadon, H., Bone, H. K., and Welham, M. J. (2004). Regulation of embryonic stem cell self-renewal by phosphoinositide 3-kinase-dependent signaling. J. Biol. Chem., 279, 48063-48070.

Pera, M. F. and Tam, P. P. L. (2010). Extrinsic regulation of pluripotent stem cells. Nature, 465, 713-720.

Rue, P. and Martinez Arias, A. (2015). Cell dynamics and gene expression control in tissue homeostasis and development. Mol. Syst. Biol., 11, 792.

Scadden, D. T. (2006). The stem-cell niche as an entity of action. Nature, 441, 1075-1079.

Schwamborn, J. C., Berezikov, E., and Knoblich, J. A. (2009). The TRIM-NHL protein TRIM32 activates microRNAs and prevents self-renewal in mouse neural progenitors. Cell, 136, 913-925.

Simons, B. D. and Clevers, H. (2011). Strategies for homeostatic stem cell self-renewal in adult tissues. Cell, 145, 851-862.

Singer, Z. S., Yong, J., Tischler, J., Hackett, J. A., Altinok, A., Surani, M. A., Cai, L., and Elowitz, M. B. (2014). Dynamic heterogeneity and DNA methylation in embryonic stem cells. Molecular cell, 55, 319-331.

Singh, A. M., Hamazaki, T., Hankowski, K. E., and Terada, N. (2007). A heterogeneous expression pattern for Nanog in embryonic stem cells. Stem Cells, 25, 2534-2542.

Sladitschek, H. L. and Neveu, P. A. (2015). MXS-Chaining: A Highly Efficient Cloning Platform for Imaging and Flow Cytometry Approaches in Mammalian Systems. PloS one, 10, e0124958.

Sonda, N., Simonato, F., Peranzoni, E., Calì, B., Bortoluzzi, S., Bisognin, A., Wang, E., Marincola, F. M., Naldini, L., Gentner, B., et al. (2013). miR-142-3p prevents macrophage differentiation during cancer induced myelopoiesis. Immunity, 38, 1236-1249.

Süel, G. M., Garcia-Ojalvo, J., Liberman, L. M., and Elowitz, M. B. (2006). An excitable gene regulatory circuit induces transient cellular differentiation. Nature, 440, 545-550.

Torres, J., Prieto, J., Durupt, F. C., Broad, S., and Watt, F. M. (2012). Efficient differentiation of embryonic stem cells into mesodermal precursors by BMP, retinoic acid and Notch signalling. PloS one, 7, e36405.

Toyooka, Y., Shimosato, D., Murakami, K., Takahashi, K., and Niwa, H. (2008). Identification and characterization of subpopulations in undifferentiated ES cell culture. Development, 135, 909-918.

Voog, J. and Jones, D. L. (2010). Stem cells and the niche: a dynamic duo. Cell Stem Cell, 6, 103-115.

Wang, Y., Medvid, R., Melton, C., Jaenisch, R., and Blelloch, R. (2007). DGCR8 is essential for microRNA biogenesis and silencing of embryonic stem cell self-renewal. Nat. Genet., 39, 380-385.

Wray, J., Kalkan, T., Gomez-Lopez, S., Eckardt, D., Cook, A., Kemler, R., and Smith, A. (2011). Inhibition of glycogen synthase kinase-3 alleviates Tcf3 repression of the pluripotency network and increases embryonic stem cell resistance to differentiation. Nature cell biology, 13, 838-845.

Yi, R., Poy, M. N., Stoffel, M., and Fuchs, E. (2008). A skin microRNA promotes differentiation by repressing 'stemness'. Nature, 452, 225-229.

Ying, Q.-L., Nichols, J., Chambers, I., and Smith, A. (2003a). BMP induction of Id proteins suppresses differentiation and sustains embryonic stem cell self-renewal in collaboration with STAT3. Cell, 115, 281-292.

Ying, Q.-L., Stavridis, M., Griffiths, D., Li, M., and Smith, A. (2003b). Conversion of embryonic stem cells into neuroectodermal precursors in adherent monoculture. Nature Biotechnol., 21, 183-186.

Ying, Q.-L., Wray, J., Nichols, J., Batlle-Morera, L., Doble, B., Woodgett, J., Cohen, P., and Smith, A. (2008). The ground state of embryonic stem cell self-renewal. Nature, 453, 519-523.

## Claims

1. A method for detecting a pluripotent stem cell (PSC) in an undifferentiated state, comprising analysing miRNA 142 expression in a PSC, wherein an increase of the expression level of miRNA 142 is indicative for an undifferentiated state, when compared to a differentiating or differentiated PSC.

2. The method according to claim 1, wherein said miRNA 142 is selected from miR-142-3p and miR-142-5p.

3. The method according to claim 1 or 2, wherein said undifferentiated PSC is a pluripotent stem cell, optionally staining positive for the pluripotency marker OCT4.

4. The method according to any one of claims 1 to 3, wherein said PSC is a mammalian PSC, such as a human, rat, or mouse PSC.

5. The method according to any one of claims 1 to 4, wherein said PSC is present in a biological sample.

6. A method for maintaining a pluripotent stem cell (PSC) in an undifferentiated state, comprising expressing or overexpressing miRNA 142 in said stem cell.

7. A method for maintaining a pluripotent stem cell (PSC) in an undifferentiated state, comprising promoting the expression or overexpression of miRNA 142 in said stem cell.

8. The method according to claim 7, wherein said miRNA 142 is selected from miR-142-3p and miR-142-5p.

9. The method according to claim 7 or 8, wherein said promoting comprises introducing an inducer of the expression of miRNA 142, an expression vector and/or an oligonucleotide comprising a region that is identical to the nucleotides of miR-142 into said PSC.

10. A method for inducing or promoting the differentiation of a pluripotent stem cell (PSC), comprising inhibiting or reducing the expression or function of miRNA 142 in said stem cell.

11. The method according to claim 10, wherein said miRNA 142 is selected from miR-142-3p and miR-142-5p.

12. The method according to claim 10 or 11, wherein said inhibiting or reducing comprises introducing an inhibitor of the expression or function of miRNA 142 into said PSC, such as, for example an oligonucleotide comprising a region that is complementary to the nucleotides of miR-142.

13. An in vitro method for identifying a compound that modulates the differentiation of a pluripotent stem cell (PSC), comprising

    a) contacting a PSC with a potential modulator compound, and
    b) detecting the expression and/or function of miRNA 142 in said PSC in response to said modulator compound,

    wherein a change in the expression and/or function of miRNA 142 is indicative for a compound that modulates the differentiation in said PSC.

14. The method according to claim 13, wherein said modulator either induces or reduces the expression and/or function of miRNA 142 in said PSC.

15. A pharmaceutical composition comprising a compound promoting and/or inducing the expression and/or function and/or overexpression of the miRNA 142 in a cell for use in medicine, in particular for use in the treatment of cancer, in particular of cancer stem cells.

**Figure 1**

**Figure 2**

## Figure 3

Figure 4

**Figure 5**

Figure 6

Figure 7

Europäisches
Patentamt

European
Patent Office

Office européen
des brevets

**EUROPEAN SEARCH REPORT**

Application Number

EP 15 20 0521

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SITI RAZILA ABDUL RAZAK ET AL: "DNA Methylation Is Involved in the Expression of miR-142-3p in Fibroblasts and Induced Pluripotent Stem Cells", STEM CELLS INTERNATIONAL, vol. 6, no. 8, 2 December 2014 (2014-12-02), pages 1290-8, XP055276826, US ISSN: 1687-966X, DOI: 10.1242/dev.105908 * paragraph [03.1]; figure 1(a)(i) * * page 2, left-hand column, paragraph 1 * | 1-5,13, 14 | INV. C12Q1/68 C12N15/11 C12N5/0735 |
| Y | WO 2004/009758 A2 (NANODIAGNOSTICS INC [IL]; SELIGMAN JUDITH [IL]) 29 January 2004 (2004-01-29) * claims 1, 11 * | 1-5 | |
| Y | WO 2011/101550 A1 (SUOMEN PUNAINEN RISTI VERIPALVELU [FI]; NATUNEN SUVI [FI]; ANDERSON HE) 25 August 2011 (2011-08-25) * claim 10 * | 13,14 | |
| X | WO 2014/152932 A1 (UNIV TEXAS [US]) 25 September 2014 (2014-09-25) * claim 39; example 7 * | 15 | TECHNICAL FIELDS SEARCHED (IPC) C12Q C12N |
| X | EP 2 505 645 A1 (TAIPEI VETERANS GENERAL HOSPITAL [TW]) 3 October 2012 (2012-10-03) * claim 5; examples 4, 5 * | 15 | |
| A | WO 2012/046065 A2 (OMNICYTE LTD [GB]; GORDON MYRTLE [GB]) 12 April 2012 (2012-04-12) * claims 1, 6 * | 6-9 | |

-/--

The present search report has been drawn up for all claims

| Place of search | Date of completion of the search | Examiner |
|---|---|---|
| The Hague | 7 June 2016 | Santagati, Fabio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| Europäisches Patentamt European Patent Office Office européen des brevets | **EUROPEAN SEARCH REPORT** | Application Number EP 15 20 0521 |
|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | A. BARROSO-DELJESUS ET AL: "The Nodal inhibitor Lefty is negatively modulated by the microRNA miR-302 in human embryonic stem cells", THE FASEB JOURNAL, vol. 25, no. 5, 1 May 2011 (2011-05-01), pages 1497-1508, XP055278025, US ISSN: 0892-6638, DOI: 10.1096/fj.10-172221 * figures 5C,D * | 6-12 | |
| A | POURRAJAB FATEMEH ET AL: "MicroRNA-based system in stem cell reprogramming; differentiation/dedifferentiation", INTERNATIONAL JOURNAL OF BIOCHEMISTRY AND CELL BIOLOGY, PERGAMON, GB, vol. 55, 20 August 2014 (2014-08-20), pages 318-328, XP029020015, ISSN: 1357-2725, DOI: 10.1016/J.BIOCEL.2014.08.008 | 1-14 | |
| A | A. JOUNEAU ET AL: "Naive and primed murine pluripotent stem cells have distinct miRNA expression profiles", RNA, vol. 18, no. 2, 27 December 2011 (2011-12-27), pages 253-264, XP055277820, US ISSN: 1355-8382, DOI: 10.1261/rna.028878.111 | 1-14 | TECHNICAL FIELDS SEARCHED (IPC) |

The present search report has been drawn up for all claims

| Place of search | Date of completion of the search | Examiner |
|---|---|---|
| The Hague | 7 June 2016 | Santagati, Fabio |

page 2 of 2

## ANNEX TO THE EUROPEAN SEARCH REPORT
## ON EUROPEAN PATENT APPLICATION NO.

EP 15 20 0521

This annex lists the patent family members relating to the patent documents cited in the above-mentioned European search report.
The members are as contained in the European Patent Office EDP file on
The European Patent Office is in no way liable for these particulars which are merely given for the purpose of information.

07-06-2016

| Patent document cited in search report | | Publication date | Patent family member(s) | | Publication date |
|---|---|---|---|---|---|
| WO 2004009758 | A2 | 29-01-2004 | AU | 2003247135 A1 | 09-02-2004 |
| | | | CA | 2493768 A1 | 29-01-2004 |
| | | | EP | 1546346 A2 | 29-06-2005 |
| | | | US | 2005287547 A1 | 29-12-2005 |
| | | | WO | 2004009758 A2 | 29-01-2004 |
| WO 2011101550 | A1 | 25-08-2011 | NONE | | |
| WO 2014152932 | A1 | 25-09-2014 | US | 2016022728 A1 | 28-01-2016 |
| | | | WO | 2014152932 A1 | 25-09-2014 |
| EP 2505645 | A1 | 03-10-2012 | CN | 102813940 A | 12-12-2012 |
| | | | EP | 2505645 A1 | 03-10-2012 |
| | | | TW | 201250005 A | 16-12-2012 |
| | | | US | 2012255043 A1 | 04-10-2012 |
| | | | US | 2013108691 A1 | 02-05-2013 |
| WO 2012046065 | A2 | 12-04-2012 | EP | 2625267 A2 | 14-08-2013 |
| | | | US | 2013217125 A1 | 22-08-2013 |
| | | | WO | 2012046065 A2 | 12-04-2012 |

EPO FORM P0459

For more details about this annex : see Official Journal of the European Patent Office, No. 12/82

## REFERENCES CITED IN THE DESCRIPTION

### Patent documents cited in the description

- US 20140288149 A
- WO 2014140856 A
- US 20140336239 A
- WO 2010056737 A
- US 20100299771 A
- US 20120004283 A
- US 20110166198 A
- US 8017763 B
- US 8173611 B
- WO 2005013901 A
- US 20120184596 A
- US 2009270481 A
- EP 1984382 A
- EP 1824975 A
- US 7834170 B
- WO 2012149646 A

### Non-patent literature cited in the description

- **ABDUL RAZAK SR ; BABA Y ; NAKAUCHI H ; OTSU M ; WATANABE S.** DNA Methylation Is Involved in the Expression of miR-142-3p in Fibroblasts and Induced Pluripotent Stem Cells. *Stem Cells Int. 2014,* 02 December 2014, 101349
- **XINYAN LU et al.** miR-142-3p regulates the formation and differentiation of hematopoietic stem cells. *vertebrates Cell Research,* 29 October 2013, vol. 23, 1356-1368
- **SHENDE VR ; NEUENDORFF N ; EARNEST DJ.** Role of miR-142-3p in the Post-Transcriptional Regulation of the Clock Gene Bmal1 in the Mouse SCN. *PLoS ONE,* 2013, vol. 8 (6), 65300
- *Cellular & Molecular Immunology,* 2014, vol. 11, 17-24
- **VIMAL K. SINGH ; MANISHA KALSAN ; NEERAJ KUMAR ; ABHISHEK SAINI ; RAMESH CHANDRA.** Induced pluripotent stem cells: applications in regenerative medicine, disease modeling, and drug discovery. *Front Cell Dev Biol.,* 2015, vol. 3, 2
- **XIANG et al.** *Nature Biotech.,* 2006, vol. 24, 697-702
- **SENZER et al.** *Mol. Therap.,* 2012, vol. 20, 679-686
- *Discov Med.,* January 2015, vol. 19 (102), 49-57
- **XIE J ; BURT DR ; GAO G.** Adeno-associated virus-mediated microRNA delivery and therapeutics. *Semin Liver Dis.,* February 2015, vol. 35 (1), 81-8
- **WANG H ; JIANG Y ; PENG H ; CHEN Y ; ZHU P ; HUANG Y.** Recent progress in microRNA delivery for cancer therapy by non-viral synthetic vectors. *Adv Drug Deliv Rev.,* January 2015, vol. 81, 142-60
- **ATTAR R ; SAJJAD F ; QURESHI MZ ; TAHIR F ; HUSSAIN E ; FAYYAZ S ; FAROOQI AA.** TRAIL based therapy: overview of mesenchymal stem cell based delivery and miRNA controlled expression of TRAIL. *Asian Pac J Cancer Prev.,* 2014, vol. 15 (16), 6495-7
- **TAY FC ; LIM JK ; ZHU H ; HIN LC ; WANG S.** Using artificial microRNA sponges to achieve microRNA loss-of-function in cancer cells. *Adv Drug Deliv Rev.,* January 2015, vol. 81, 117-27
- **LEMOINNE S ; THABUT D ; HOUSSET C ; MOREAU R ; VALLA D ; BOULANGER CM ; RAUTOU PE.** The emerging roles of microvesicles in liver diseases. *Nat Rev Gastroenterol Hepatol.,* June 2014, vol. 11 (6), 350-61
- **BREVING et al.** *Int J Biochem Cell Biol.,* August 2010, vol. 42 (8), 1316-29
- **VAN ROOIJ et al.** *Circ Res.,* 03 February 2012, vol. 110 (3), 496-507
- **IORIO et al.** *EMBO Mol. Med.,* March 2012, vol. 4 (3), 143-159
- **FRIEDEN, M. et al.** *Curr. Pharm. Des.,* 2008, vol. 14 (11), 1138-1142
- **TABATA et al.** Multiparametric Phenotypic Screening System for Profiling Bioactive Compounds Using Human Fetal Hippocampal Neural Stem/Progenitor Cells. *J Biomol Screen.,* October 2015, vol. 20 (9), 1074-83
- **BARTEL, D. P.** MicroRNAs: target recognition and regulatory functions. *Cell,* 2009, vol. 136, 215-233
- **BENJAMINI, Y. ; HOCHBERG, Y.** Controlling the false discovery rate: a practical and powerful approach to multiple testing. *J. R. Stat. Soc. B,* 1995, vol. 57, 289-300

- **BLANPAIN, C. ; FUCHS, E.** Stem cell plasticity. Plasticity of epithelial stem cells in tissue regeneration. *Science,* 2014, vol. 344, 1242281
- **BOROWIAK, M. ; MAEHR, R. ; CHEN, S. ; CHEN, A. E. ; TANG, W. ; FOX, J. L. ; SCHREIBER, S. L. ; MELTON, D. A.** Small molecules efficiently direct endodermal differentiation of mouse and human embryonic stem cells. *Cell Stem Cell,* 2009, vol. 4, 348-358
- **BURDON, T. ; STRACEY, C. ; CHAMBERS, I. ; NICHOLS, J. ; SMITH, A.** Suppression of SHP-2 and ERK signalling promotes self-renewal of mouse embryonic stem cells. *Dev. Biol.,* 1999, vol. 210, 30-43
- **CANHAM, M. A. ; SHAROV, A. A. ; KO, M. S. H. ; BRICKMAN, J. M.** Functional heterogeneity of embryonic stem cells revealed through translational amplification of an early endodermal transcript. *PLoS Biol.,* 2010, vol. 8, 1000379
- **CHAMBERS, I. ; SILVA, J. ; COLBY, D. ; NICHOLS, J. ; NIJMEIJER, B. ; ROBERTSON, M. ; VRANA, J. ; JONES, K. ; GROTEWOLD, L. ; SMITH, A.** Nanog safeguards pluripotency and mediates germline development. *Nature,* 2007, vol. 450, 1230-1234
- **CHAPNIK, E. ; RIVKIN, N. ; MILDNER, A. ; BECK, G. ; PASVOLSKY, R. ; METZL-RAZ, E. ; BIRGER, Y. ; AMIR, G. ; TIROSH, I. ; PORAT, Z. et al.** miR-142 orchestrates a network of actin cytoskeleton regulators during megakaryopoiesis. *eLife,* 2014, vol. 3, 01964
- **CHEN, C.-Z. ; LI, L. ; LODISH, H. F. ; BARTEL, D. P.** MicroRNAs modulate hematopoietic lineage differentiation. *Science,* 2004, vol. 303, 83-86
- **COHEN, D. E. ; MELTON, D.** Turning straw into gold: directing cell fate for regenerative medicine. *Nat. Rev. Genet.,* 2011, vol. 12, 243-252
- **EVANS, M. J. ; KAUFMAN, M. H.** Establishment in culture of pluripotential cells from mouse embryos. *Nature,* 1981, vol. 292, 154-156
- **FORONDA, D. ; WENG, R. ; VERMA, P. ; CHEN, Y.-W. ; COHEN, S. M.** Coordination of insulin and Notch pathway activities by microRNA miR-305 mediates adaptive homeostasis in the intestinal stem cells of the Drosophila gut. *Genes Dev,* 2014, vol. 28, 2421-2431
- **GREBER, B. ; WU, G. ; BERNEMANN, C. ; JOO, J. Y. ; HAN, D. W. ; KO, K. ; TAPIA, N. ; SABOUR, D. ; STERNECKERT, J. ; TESAR, P. et al.** Conserved and divergent roles of FGF signaling in mouse epiblast stem cells and human embryonic stem cells. *Cell Stem Cell,* 2010, vol. 6, 215-226
- **GRIFFITHS-JONES, S. ; SAINI, H. K. ; VAN DONGEN, S. ; ENRIGHT, A. J.** miRBase: tools for microRNA genomics. *Nucleic Acids Res.,* 2008, vol. 36, 154-8
- **GRIMSON, A. ; FARH, K. K.-H. ; JOHNSTON, W. K. ; GARRETT-ENGELE, P. ; LIM, L. P. ; BARTEL, D. P.** MicroRNA targeting specificity in mammals: determinants beyond seed pairing. *Mol. Cell,* 2007, vol. 27, 91-105
- **GUPTA, P. B. ; FILLMORE, C. M. ; JIANG, G. ; SHAPIRA, S. D. ; TAO, K. ; KUPERWASSER, C. ; LANDER, E. S.** Stochastic state transitions give rise to phenotypic equilibrium in populations of cancer cells. *Cell,* 2011, vol. 146, 633-644
- **HSU, P. D. ; SCOTT, D. A. ; WEINSTEIN, J. A. ; RAN, F. A. ; KONERMANN, S. ; AGARWALA, V. ; LI, Y. ; FINE, E. J. ; WU, X. ; SHALEM, O. et al.** DNA targeting specificity of RNA-guided Cas9 nucleases. *Nat. Biotechnol.,* 2013, vol. 31, 827-832
- **JIANG, H. ; PATEL, P. H. ; KOHLMAIER, A. ; GRENLEY, M. O. ; MCEWEN, D. G. ; EDGAR, B. A.** Cytokine/Jak/Stat signaling mediates regeneration and homeostasis in the Drosophila midgut. *Cell,* 2009, vol. 137, 1343-1355
- **JOHNSON, L. ; GREENBAUM, D. ; CICHOWSKI, K. ; MERCER, K. ; MURPHY, E. ; SCHMITT, E. ; BRONSON, R. ; UMANOFF, H. ; EDELMANN, W. ; KUCHERLAPATI, R. et al.** K-ras is an essential gene in the mouse with partial functional overlap with n-ras. *Genes & Development,* 1997, vol. 11, 2468-2481
- **JOHNSTON, R. J. ; CHANG, S. ; ETCHBERGER, J. F. ; ORTIZ, C. O. ; HOBERT, O.** MicroRNAs acting in a double-negative feedback loop to control a neuronal cell fate decision. *Proc. Natl Acad. Sci. USA,* 2005, vol. 102, 12449-12454
- **KHOKHLATCHEV, A. V. ; CANAGARAJAH, B. ; WILSBACHER, J. ; ROBINSON, M. ; ATKINSON, M. ; GOLD-SMITH, E. ; COBB, M. H.** Phosphorylation of the MAP kinase ERK2 promotes its homodimerization and nuclear translocation. *Cell,* 1998, vol. 93, 605-615
- **KLEIN, A. M. ; NAKAGAWA, T. ; ICHIKAWA, R. ; YOSHIDA, S. ; SIMONS, B. D.** Mouse germ line stem cells undergo rapid and stochastic turnover. *Cell Stem Cell,* 2010, vol. 7, 214-224
- **KRAMER, N. J. ; WANG, W.-L. ; REYES, E. Y. ; KUMAR, B. ; CHEN, C.-C. ; RAMAKRISHNA, C. ; CANTIN, E. M. ; VONDERFECHT, S. L. ; TAGANOV, K. D. ; CHAU, N. et al.** Altered lymphopoiesis and immunodeficiency in miR-142 null mice. *Blood,* 2015, vol. 125, 3720-3730
- **KUEH, H. Y. ; CHAMPHEKAR, A. ; CHAMPHEKAR, A. ; NUTT, S. L. ; ELOWITZ, M. B. ; ROTHENBERG, E. V.** Positive feedback between PU.1 and the cell cycle controls myeloid differentiation. *Science,* 2013, vol. 341, 670-673

- **KUNATH, T. ; SABA-EL-LEIL, M. K. ; ALMOUSAIL-LEAKH, M. ; WRAY, J. ; MELOCHE, S. ; SMITH, A.** FGF stimulation of the Erk1/2 signalling cascade triggers transition of pluripotent embryonic stem cells from self-renewal to lineage commitment. *Development,* 2007, vol. 134, 2895-2902
- **LANGMEAD, B. ; TRAPNELL, C. ; POP, M. ; SALZBERG, S. L.** Ultrafast and memory-efficient alignment of short DNA sequences to the human genome. *Genome Biol.,* 2009, vol. 10, 25
- **LI, X. ; CARTHEW, R. W.** A microRNA mediates EGF receptor signaling and promotes photoreceptor differentiation in the Drosophila eye. *Cell,* 2005, vol. 123, 1267-1277
- **LOSICK, R. ; DESPLAN, C.** Stochasticity and cell fate. *Science,* 2008, vol. 320, 65-68
- **MARTIN, G. R.** Isolation of a pluripotent cell line from early mouse embryos cultured in medium conditioned by teratocarcinoma stem cells. *Proc. Natl. Acad. Sci. USA,* 1981, vol. 78, 7634-7638
- **MCBURNEY, M. W. ; SUTHERLAND, L. C. ; ADRA, C. N. ; LECLAIR, B. ; RUDNICKI, M. A. ; JARDINE, K.** The mouse Pgk-1 gene promoter contains an upstream activator sequence. *Nucleic Acids Res.,* 1991, vol. 19, 5755-5761
- **MEDEMA, J. P ; VERMEULEN, L.** Microenvironmental regulation of stem cells in intestinal homeostasis and cancer. *Nature,* 2011, vol. 474, 318-326
- **MILDNER, A. ; CHAPNIK, E. ; MANOR, O. ; YONA, S. ; KIM, K.-W. ; AYCHEK, T. ; VAROL, D. ; BECK, G. ; ITZHAKI, Z. B. ; FELDMESSER, E. et al.** Mononuclear phagocyte miRNome analysis identifies miR-142 as critical regulator of murine dendritic cell homeostasis. *Blood,* 2013, vol. 121, 1016-1027
- **MURRY, C. E. ; KELLER, G.** Differentiation of embryonic stem cells to clinically relevant populations: lessons from embryonic development. *Cell,* 2008, vol. 132, 661-680
- **NEUMÜLLER, R. A. ; BETSCHINGER, J. ; FISCHER, A. ; BUSHATI, N. ; POERNBACHER, I. ; MECHTLER, K. ; COHEN, S. M. ; KNOBLICH, J. A.** Mei-P26 regulates microRNAs and cell growth in the Drosophila ovarian stem cell lineage. *Nature,* 2008, vol. 454, 241-245
- **NEVEU, P. ; KYE, M. J. ; QI, S. ; BUCHHOLZ, D. E. ; CLEGG, D. O. ; SAHIN, M. ; PARK, I.-H. ; KIM, K.-S. ; DALEY, G. Q. ; KORNBLUM, H. I. et al.** MicroRNA profiling reveals two distinct p53-related human pluripotent stem cell states. *Cell Stem Cell,* 2010, vol. 7, 671-681
- **NIMMO, R. ; CIAU-UITZ, A. ; RUIZ-HERGUIDO, C. ; SONEJI, S. ; BIGAS, A. ; PATIENT, R. ; ENVER, T.** miR-142-3p controls the specification of definitive hemangioblasts during ontogeny. *Dev. Cell,* 2013, vol. 26, 237-249
- **NIWA, H. ; BURDON, T. ; CHAMBERS, I. ; SMITH, A.** Self-renewal of pluripotent embryonic stem cells is mediated via activation of STAT3. *Genes Dev.,* 1998, vol. 12, 2048-2060
- **NIWA, H. ; YAMAMURA, K. ; MIYAZAKI, J.** Efficient selection for high-expression transfectants with a novel eukaryotic vector. *Gene,* 1991, vol. 108, 193-199
- **NORTH, T. E. ; GOESSLING, W. ; WALKLEY, C. R. ; LENGERKE, C. ; KOPANI, K. R. ; LORD, A. M. ; WEBER, G. J. ; BOWMAN, T. V. ; JANG, I. H. ; GROSSER, T. et al.** Prostaglandin E2 regulates vertebrate haematopoietic stem cell homeostasis. *Nature,* 2007, vol. 447, 1007-1011
- **PALING, N. R. D. ; WHEADON, H. ; BONE, H. K. ; WELHAM, M. J.** Regulation of embryonic stem cell self-renewal by phosphoinositide 3-kinase-dependent signaling. *J. Biol. Chem.,* 2004, vol. 279, 48063-48070
- **PERA, M. F. ; TAM, P. P. L.** Extrinsic regulation of pluripotent stem cells. *Nature,* 2010, vol. 465, 713-720
- **RUE, P. ; MARTINEZ ARIAS, A.** Cell dynamics and gene expression control in tissue homeostasis and development. *Mol. Syst. Biol.,* 2015, vol. 11, 792
- **SCADDEN, D. T.** The stem-cell niche as an entity of action. *Nature,* 2006, vol. 441, 1075-1079
- **SCHWAMBORN, J. C. ; BEREZIKOV, E. ; KNOBLICH, J. A.** The TRIM-NHL protein TRIM32 activates microRNAs and prevents self-renewal in mouse neural progenitors. *Cell,* 2009, vol. 136, 913-925
- **SIMONS, B. D. ; CLEVERS, H.** Strategies for homeostatic stem cell self-renewal in adult tissues. *Cell,* 2011, vol. 145, 851-862
- **SINGER, Z. S. ; YONG, J. ; TISCHLER, J. ; HACKETT, J. A. ; ALTINOK, A. ; SURANI, M. A. ; CAI, L. ; ELOWITZ, M. B.** Dynamic heterogeneity and DNA methylation in embryonic stem cells. *Molecular cell,* 2014, vol. 55, 319-331
- **SINGH, A. M. ; HAMAZAKI, T. ; HANKOWSKI, K. E. ; TERADA, N.** A heterogeneous expression pattern for Nanog in embryonic stem cells. *Stem Cells,* 2007, vol. 25, 2534-2542
- **SLADITSCHEK, H. L. ; NEVEU, P. A.** MXS-Chaining: A Highly Efficient Cloning Platform for Imaging and Flow Cytometry Approaches in Mammalian Systems. *PloS one,* 2015, vol. 10, 0124958
- **SONDA, N. ; SIMONATO, F. ; PERANZONI, E. ; CALÌ, B. ; BORTOLUZZI, S. ; BISOGNIN, A. ; WANG, E. ; MARINCOLA, F. M. ; NALDINI, L. ; GENTNER, B. et al.** miR-142-3p prevents macrophage differentiation during cancer induced myelopoiesis. *Immunity,* 2013, vol. 38, 1236-1249

- **SÜEL, G. M. ; GARCIA-OJALVO, J. ; LIBERMAN, L. M. ; ELOWITZ, M. B.** An excitable gene regulatory circuit induces transient cellular differentiation. *Nature,* 2006, vol. 440, 545-550
- **TORRES, J. ; PRIETO, J. ; DURUPT, F. C. ; BROAD, S. ; WATT, F. M.** Efficient differentiation of embryonic stem cells into mesodermal precursors by BMP, retinoic acid and Notch signalling. *PloS one,* 2012, vol. 7, 36405
- **TOYOOKA, Y. ; SHIMOSATO, D. ; MURAKAMI, K. ; TAKAHASHI, K. ; NIWA, H.** Identification and characterization of subpopulations in undifferentiated ES cell culture. *Development,* 2008, vol. 135, 909-918
- **VOOG, J. ; JONES, D. L.** Stem cells and the niche: a dynamic duo. *Cell Stem Cell,* 2010, vol. 6, 103-115
- **WANG, Y. ; MEDVID, R. ; MELTON, C. ; JAENISCH, R. ; BLELLOCH, R.** DGCR8 is essential for microRNA biogenesis and silencing of embryonic stem cell self-renewal. *Nat. Genet.,* 2007, vol. 39, 380-385
- **WRAY, J. ; KALKAN, T. ; GOMEZ-LOPEZ, S. ; ECKARDT, D. ; COOK, A. ; KEMLER, R. ; SMITH, A.** Inhibition of glycogen synthase kinase-3 alleviates Tcf3 repression of the pluripotency network and increases embryonic stem cell resistance to differentiation. *Nature cell biology,* 2011, vol. 13, 838-845
- **YI, R. ; POY, M. N. ; STOFFEL, M. ; FUCHS, E.** A skin microRNA promotes differentiation by repressing 'stemness. *Nature,* 2008, vol. 452, 225-229
- **YING, Q.-L. ; NICHOLS, J. ; CHAMBERS, I. ; SMITH, A.** BMP induction of Id proteins suppresses differentiation and sustains embryonic stem cell self-renewal in collaboration with STAT3. *Cell,* 2003, vol. 115, 281-292
- **YING, Q.-L. ; STAVRIDIS, M. ; GRIFFITHS, D. ; LI, M. ; SMITH, A.** Conversion of embryonic stem cells into neuroectodermal precursors in adherent monoculture. *Nature Biotechnol.,* 2003, vol. 21, 183-186
- **YING, Q.-L. ; WRAY, J. ; NICHOLS, J. ; BATLLE-MORERA, L. ; DOBLE, B. ; WOODGETT, J. ; COHEN, P. ; SMITH, A.** The ground state of embryonic stem cell self-renewal. *Nature,* 2008, vol. 453, 519-523

SEQUENCE LISTING

<110>  European Molecular Biology Laboratory

<120>  microRNA miR-142 as stem cell marker

<130>  E31812EP

<160>  10

<170>  PatentIn version 3.5

<210>  1
<211>  21
<212>  RNA
<213>  Homo sapiens

<400>  1
cauaaaguag aaagcacuac u                                                    21


<210>  2
<211>  23
<212>  RNA
<213>  Homo sapiens

<400>  2
uguaguguuu ccuacuuuau gga                                                  23


<210>  3
<211>  87
<212>  RNA
<213>  Homo sapiens

<400>  3
gacagugcag ucacccauaa aguagaaagc acuacuaaca gcacuggagg guguaguguu          60

uccuacuuua uggaugagug uacugug                                              87


<210>  4
<211>  111
<212>  DNA
<213>  Homo sapiens

<400>  4
gcgtgatctc cgaagcccac agtacactca tccataaagt aggaaacact acaccctcca          60

gtgctgttag tagtgctttc tactttatgg gtgactgcac tgtctgtctg t                  111


<210>  5
<211>  20
<212>  DNA
<213>  Mus musculus

<400>  5
ggtggcctga agaatccccg                                                      20


<210>  6
<211>  20

```
<212>   DNA
<213>   Mus musculus

<400>   6
ggagccatga aggtctttcg                                          20


<210>   7
<211>   20
<212>   DNA
<213>   Mus musculus

<400>   7
tatactcagt cattttcagc                                          20


<210>   8
<211>   20
<212>   DNA
<213>   Mus musculus

<400>   8
gactgagtat aaacttgtgg                                          20


<210>   9
<211>   20
<212>   DNA
<213>   Mus musculus

<400>   9
ctgaattagc tgtatcgtca                                          20


<210>   10
<211>   20
<212>   DNA
<213>   Mus musculus

<400>   10
gctaattcag aatcactttg                                          20
```